# EUROPÄISCHE PATENTANMELDUNG

(11) **EP 2 720 198 A2**
(43) Veröffentlichungstag der Anmeldung: **16.04.2014**
(21) Anmeldenummer: 13004894.5
(22) Anmeldetag: 11.10.2013
(51) Int. Cl.: G07C 9/00

(54) **Kontrollvorrichtung zur Kontrolle des Transfers von Wäschestücken aus bzw. in ein Wäschemagazin**

(30) Priorität: 15.10.2012 DE 102012020125
(71) Anmelder: LEO System GmbH, 20354 Hamburg (DE)
(72) Erfinder: Fastenau, Andreas, 58332 Schwelm (DE); Schöppe, Sven, 58089 Hagen (DE)
(74) Vertreter: Wagner, Carsten

(57) **Zusammenfassung**

Die Erfindung betrifft eine Kontrollvorrichtung zur Kontrolle des Transfers von Wäschestücken aus bzw. in ein Wäschemagazin.Dazu weist diese eine Zugangseinrichtung (4), die wiederum in Verwendungsposition einen für einen Benutzer bidirektional passierbaren Zugang (6) zum Wäschemagazin (8) aufweist. Dabei ist eine Steuerungseinrichtung vorgesehen, die Mittel zur Erfassung von Benutzeridentifikationsdaten des Benutzers (10) aufweist, um mittels der erfassten Benutzeridentifikationsdaten eine Zugangspassierberechtigung des Benutzer (10) für ein Passieren des Zugangs (12, 12') zu ermitteln, so dass die Steuerungseinrichtung (14) ein Sperrorgan (18,18') der Zugangseinrichtung (8) in Abhängigkeit der Zugangspassierberechtigung des Benutzers (10) zwischen einem Sperrzustand, in dem der Zugang durch das Sperrorgan (18,18') zum Passieren gesperrt ist, und einem Entsperrzustand, in dem der Zugang (12) durch das Sperrorgan (18,18') zum Passieren entsperrt ist, steuert. Dazu weist die Steuerungseinrichtung (14) erste Erfassungsmittel (14) auf, die zur berührungslosen Erfassung von Wäschestückidentifikationsdaten einzelner Wäschestücke (4) aus einer Mehrzahl in wahlfreier Anordnung zueinander und wahlfreier Zusammenstellung den Zugang passierender Wäschestücke (4) eingerichtet und ausgebildet sind, wobei die Steuerungseinrichtung die erfassten Wäschestückidentifikationsdaten den erfassten Benutzeridentifikationsdaten zuordnet und diese Zuordnung in einem Datenspeicher (22) verwaltet.

## Beschreibung

Die Erfindung betrifft gemäß dem Oberbegriff des Anspruchs 1 eine Kontrollvorrichtung für Räume, die der Kontrolle des Transfers von Wäschestücken aus bzw. in ein Wäschemagazin dient.

Für Kontrollen des Transfers von Wäschestücken aus bzw. in ein Wäschemagazin existieren verschiedene Möglichkeiten, die von einer manuellen bis zu einer automatisierten Verwaltung reichen.

Eine manuelle Kontrolle des Transfers von Wäschestücken aus bzw. in ein Wäschemagazin eines gewerblichen Betriebs bzw. einer vergleichbaren Einrichtung erfolgt u.a. dadurch, dass ausgegebenen Wäschestücke von einer entsprechend autorisierten Person dokumentiert wird.

Die Berechtigung zum Erhalt der Wäschestücke wird dabei von der autorisierten Person beispielsweise anhand eines Dienstausweises der betreffenden Person geprüft und ggf. mit von Art und Anzahl der erhalten Wäschestücke handschriftlich wie auch rechnergestützt dokumentiert. Dabei unterbleibt dabei häufig eine Dokumentation der von der Person retournierten Wäschestücke. Verbreitet ist auch eine Selbstkontrolle der Mitarbeiter, so dass ein Wäschemagazin dem Personal einer Einrichtung frei zugänglich ist.

Der Zugang zu einem Wäschemagazin erfolgt dabei unter anderem durch eine sperrbare/entsperrbare Tür, die beispielsweise zum Zwecke ihres Öffnens über einen Zugangscode entsperrt werden kann. Dazu werden unter anderem maschinenlesbare Identifikationsmedien (z.B. eine maschinenlesbare Identifikationskarte) verwendet, wobei eine Anmeldung mittels einer solchen Identifikationsmediums durch den Benutzer an einem Terminal als Bestandteil einer Steuerungseinrichtung erfolgen kann.

Aufgrund der auf diesem Identifikationsmedium gespeicherten Daten ermittelt eine Steuerungseinrichtung eine Zugangspassierberechtigung des Benutzers für das Passieren des Zugangs in das bzw. aus dem Wäschemagazin und steuert daraufhin die Tür von einem Sperrzustand, in dem der Zugang in das Wäschemagazin durch die Tür bzw. ein Sperrorgan für ein Passieren der Person bzw. von Kleidungsstücken gesperrt ist, in einen Entsperrzustand, in dem der Zugang durch das Sperrorgan für ein Passieren der Person bzw. von Kleidungsstücken entsperrt ist.

Nachteilig bei einer manuell durchgeführte Kontrolle eines Wäschemagazins ist der hohe Personalbedarf bzw. ein erhöhtes Risiko von Fehlern der Vorgangsdokumentation. Ferner ist bei Reduzierung des Personalbedarfs nachteilig, dass eine Entnahme von Wäschestücken aus dem Wäschemagazin ggf. nur zu festgelegten Zeiten erfolgt, woraufhin sich eine Einschränkung in der Verfügbarkeit sauberer Wäschestücke ergeben kann.

Darüber hinaus sind automatisierte Einrichtungen bekannt, die die Kontrolle eines Wäschemagazins unterstützen bzw. vollautomatisiert vornehmen. Dadurch ist der personelle Aufwand für das Betreiben eines Wäschemagazins verringerbar. Gleichsam ist unter Verwendung einer automatisierten Einrichtungen ein zeitlich uneingeschränkter Zugriff zu entsprechenden Wäschestücken bzw. Kleidungsstücken ermöglicht.

Dazu existieren verschiedene Systeme, die den Benutzer das gewünschte Wäschestück automatisch ausgeben. Beispielsweise prüft eine der bekannten Kontrolleinrichtungen zunächst die Berechtigung des Benutzers zum Erhalt eines Kleidungsstückes. Dies erfolgt z.B. mittels einer Codeeingabe oder einer sogenannten Chipkarte, auf der die zur Ermittlung einer Zugangspassierberechtigung notwendigen Daten gespeichert sind.

Bei vorliegender Berechtigung wählt der Benutzer mittels einer Eingabe ein Kleidungsstück aus, dass anschließend über eine Ausgabe dem Benutzer bereitgestellt wird. Derartige Ausgabeautomaten können nach Art eines Getränkeautomaten aufgebaut sein und aufgrund ihrer kompakten Bauweise und eines autarken Betriebsmöglichkeit an verschiedenen Orten eines Betriebs oder einer Einrichtung, in der Wäschestücke zum Wechseln bereitgestellt werden müssen, aufgestellt werden. Im einfachsten Fall reicht die Bereitstellung von Energie zum Betrieb eines solchen Ausgabeautomaten.

Ferner sind Systeme für Wäschestücke bekannt, die eine hängende Lagerung der Wäschestücke vorsehen, wobei die Wäschestücke z.B. getrennt voneinander auf Bügeln gelagert werden. Die Bügel sind an Förderanlage angeordnet und bilden einen Lagerplatz, dem ein Kleidungsstück zugeordnet wird. Derartige Förderanlagen sind beispielsweise nach Art eines Karussells ausgebildet und beispielsweise auch in Wäschereien im Einsatz. Über die Zuordnung des Kleidungsstückes zu einem Lagerplatz an der Förderanlage können die Kleidungsstücke identifiziert und entsprechend automatisiert ausgegeben werden. Die Lagerung der Kleidungsstücke kann dabei chaotisch erfolgen, so dass über ein Steuerungssystem der Lagerplatz des Wäschestückes verwaltet wird und das Wäschestück entsprechend einer Eingabe des Benutzers aufgefunden und ausgegeben werden kann. Die Einlagerung der Wäschestücke ist gegenüber einer Ablage eines Kleidungsstückes in einem Regal aufwendiger, da die Kleidungsstücke mit einem individuellen Lagerplatz der Steuerungseinrichtung in Bezug gesetzt werden und entsprechend in einem Datenspeicher verwaltete werden, damit diese aufgrund der gespeicherten Zuordnung wieder aufgefunden werden können. Ferner sind die Wäschestücke an einem entsprechenden Haltemittel, wie dies der zuvor genannte Bügel sein kann, anzuordnen.

Nachteilig sind die hohen Investitionskosten für derartige Einrichtungen. Ferner ist die Lagerung eines lediglich begrenzten Vorrates im Vergleich zu einer manuellen Lagerung bei gleichem Raumangebot möglich. Derartige Systeme erfordern für ein Aufstellen der Förderanlage ausreichend große Verkehrsflächen, damit diese eingesetzt werden können. Dies reduziert den Nutzraum zur Aufbewahrung von Wäschestücken. Ferner sind Wäschemagazine, in denen diese Systeme eingesetzt werden, während des Betriebs des Systems nicht dem Benutzer bzw. einer Personen zugänglich, woraufhin durch die Ausgabe der Wäschestücke Wartezeiten auch für weitere Benutzer entstehen können. Zudem sind z.B. bei einem Systemfehler die gewünschten Kleidungsstücke bei chaotischer Lagerung nur schwer auffindbar.

Einschränkungen der Automaten können durch eine turnusmäßig anstelle einer bedarfsgemäßen Bestückung des Wäschemagazins eintreten, wobei eine Fehlbestand eintreten kann, in dem mehr Wäschestücke als prognostiziert dem Automaten entnommen wurden. Dadurch ist eine verlässliche Deckung des Bedarfs an Wäschestücken nicht gewährleistet.

Die bekannten Systeme verleiten das Personal dazu, einen persönlicher Vorrat an Wäschestücken außerhalb des Wäschemagazins anzulegen, wodurch die Anzahl der im Umlauf befindlichen Wäschestücke über einem tatsächlichen bzw. angemessenen Bedarf liegt und damit unnötig hoch ist. Folglich müssen eine entsprechende Anzahl von Wäschestücken im Bestand bzw. im Umlauf gehalten werden, was zu einer unnötigen Kapitalbindung durch die Wäschestücken führt.

Für eine Wäschemagazin haben sich unterschiedliche Betreibermodelle etabliert, so dass Wäschestücke vielfach durch einen Wäschestückanbieter, wie es ein Reinigungsunternehmen für Wäschestücke sein kann, zur Nutzung durch das Personal eines Kunden bereitgestellt werden. Dabei entrichtet der Kunde für die Bereitstellung und Reinigung der Wäschestücke ein entsprechendes Entgelt, ohne die Wäschestücke selbst zu erwerben. Dabei binden insbesondere nicht nachvollziehbar aus einem Wäschemagazin entnommene Wäschestücke unnötig viel Kapital.

Vor diesem Hintergrund ergibt sich durch die bekannten Systeme bzw. Verfahren zur Kontrolle des Einbringens und der Entnahme von Wäschestücken ein Nachteil darin, dass die Deckung eines tatsächlichen Bedarfs an Wäschestücken nicht oder nur sehr aufwendig ermittelbar ist.

Der Erfindung liegt die Aufgabe zugrunde, eine Unterversorgung von Wäschestücken wirksam zu vermeiden, die Sensibilität hinsichtlich der Entnahme von Wäschestücken durch eine Person/ einen Mitarbeiter zu erhöhen und die Kapitalbindung der für den Gebrauch bereitgestellte Wäschestücke zu verringern. Ferner liegt der Erfindung die Aufgabe zugrunde, den Nutzraum zur Ablage der Wäschestücke in einem Wäschemagazin zu maximieren und eine Möglichkeit zu schaffen, bereits bestehende Räumlichkeiten für ein Wäschemagazin auf einfache Art und Weise durch eine Kontrollvorrichtung bezüglich des Bestands an Wäschestücken in dem Wäschemagazin mit möglichst geringem Aufwand zu ergänzen bzw. auszustatten, um die notwendigen baulichen Veränderungsmaßnahmen möglichst gering zu halten.

Die Erfindung entfernt sich von dem Gedanken, bereits bestehende Systeme beispielsweise aus der Materialwirtschaft von Industrieunternehmen für den Bereich der Wäschelogistik anzupassen, die zu hohen Investitionen für den Betreiber eines Wäschemagazins führen bzw. eine hohe Realisierungskomplexität aufweisen wie auch eine große Verkehrsfläche zu deren Einsetzbarkeit benötigen. Ferner berücksichtigt die Erfindung den Aspekt, bei einem technisch bedingten Ausfall der Kontrollvorrichtung den Betrieb des Wäschemagazins aufrechterhalten zu können, um eine Verfügbarkeit von Wäschestücken auch während möglicher Ausfallzeiten gewährleisten zu können.

Zudem entfernt sich die Erfindung von dem Gedanken, eine unnötige Überkapazität von Wäschestücken zu bilden, um Bedarfsspitzen mit einem entsprechenden Vorrat an Wäschestücken bedienen zu können.

Daher liegt der Erfindung der Gedanke zugrunde, eine Kontrollvorrichtung anzugeben, welche vergleichsweise einfach im Aufbau ist und sowohl die Zufuhr von Wäschestücken in ein Wäschemagazin wie auch die Entnahme von Wäschestücken aus einem Wäschemagazin (also den Transfer von Wäschestücken aus einem Wäschemagazin bzw. in ein Wäschemagazin) zuverlässig erfasst, um den Bestand an Wäschestücken in dem Wäschemagazin wie auch den Verbleib der im Umlauf befindlichen Wäschestück ermitteln zu können. Gleichfalls stellt sich die Erfindung der Aufgabe, eine einfache Handhabung einer Kontrollvorrichtung zu ermöglichen. Dabei verfolgt die Erfindung die Zielrichtung, die Raumnutzung zur Aufbewahrung bzw. Lagerung von Wäschestücken möglichst zu maximieren, um die für die Entnahme bzw. das Einbringen von Wäschestücken notwendige Verkehrsfläche möglich gering zu halten. Gleichfalls soll durch die Erfindung ein vollautomatisierbarer Betrieb eines Kontrollsystems, insbesondere bezüglich der Erfassung von Wäschestücken für den Transfer aus bzw. in ein Wäschemagazin, ermöglicht werden.

Die Erfindung löst die Aufgabe auf überraschend einfache Art und Weise dadurch, dass eine Kontrollvorrichtung zunächst einen Zugang zu einem Personen begehbaren Wäschemagazin bereitstellt und diesen hinsichtlich der Zugangspassierberechtigung eines Zugangs zu diesem Wäschemagazin kontrolliert.

Dabei liegt der Erfindung der Grundgedanke zugrunde, die Zugangspassierberechtigung nicht nur abhängig von insbesondere der Tätigkeit einer Person festzulegen, sondern auch von den aus dem Wäschemagazin entnommenen Wäschestücken abhängig machen zu können, die durch den Benutzer bereits aus dem Wäschemagazin entnommen wurden und somit diesem Benutzer zugeordnet werden können. Dabei berücksichtigt die Erfindung die Möglichkeit, verschiedene Kriterien für eine Zugangspassierberechtigung festlegen wie auch entsprechend kontrollieren zu können.

Erfindungsgemäß ist dabei ein Benutzer eine Person, die insbesondere zum Zwecke einer Entnahme aus dem Wäschemagazin bzw. einem Einbringen von Wäschestücken in das Wäschemagazin eine erfindungsgemäße Kontrollvorrichtung benutzt.

Die Erfindung löst die Aufgabe durch eine Kontrollvorrichtung zur Kontrolle des Transfers von Wäschestücken aus bzw. in ein Wäschemagazin.

Im Hinblick auf einen größtmöglichen Nutzraum zur Aufbewahrung von Wäschestücken sieht eine erfindungsgemäße Kontrollvorrichtung zur Kontrolle des Transfers von Wäschestücken aus bzw. in ein Wäschemagazin eine Zugangseinrichtung vor, die in Verwendungsposition, in der die Kontrollvorrichtung für die Kontrolle eines Wäschemagazins eingesetzt und verwendbar ist, einen für einen Benutzer bidirektional passierbaren Zugang zum Wäschemagazin auf. Das Wäschemagazin wird dabei zum Zwecke der Entnahme von Wäschestücken bzw. zum Einbringen von Wäschestücken in das Wäschemagazin von dem Benutzer begangen, um das gewünschte Wäschestücke einem Lagerplatz, der z.B. in einem Regal oder einem Behältnis gebildet ist, entnehmen zu können.

Dazu weist eine erfindungsgemäße Kontrollvorrichtung eine Steuerungseinrichtung auf, die wiederum Mittel zur Erfassung von Benutzeridentifikationsdaten des Benutzers aufweist, um mittels der erfassten Benutzeridentifikationsdaten eine Zugangspassierberechtigung des Benutzer für ein Passieren des Zugangs zu ermitteln. Dies führt dazu, dass die Steuerungseinrichtung wenigstens ein Sperrorgan der Zugangseinrichtung in Abhängigkeit der Zugangspassierberechtigung des Benutzers zwischen einem Sperrzustand, in dem der Zugang durch das Sperrorgan zum Passieren gesperrt ist, und einem Entsperrzustand, in dem der Zugang durch das Sperrorgan zum Passieren entsperrt ist, steuert. Dabei ist erfindungsgemäße berücksichtigt, dass Wäschestücke nicht zwingend in Begleitung einer Person den Zugang passieren müssen. Beispielsweise und insbesondere ist berücksichtigt, dass Wäschestücke auch in Gebinde mittels Transporthilfsmittel den Zugang passieren können.

Demgemäß ist erfindungsgemäß vorgesehen, dass die Steuerungseinrichtung die Zugangsvorrichtung in Abhängigkeit der ermittelten Zugangspassierberechtigung des Benutzers zum Wechsel von einem Sperrzustand in einen Entsperrzustand steuert. Dazu ist die Steuerungseinrichtung mit der Zugangsvorrichtung zur Steuerung signal- bzw. steuerungstechnisch verbunden, um beispielsweise Aktoren des Sperrorgans entsprechend anzusteuern.

Erfindungsgemäß ist dabei ein Passieren des Zugangs bezogen auf Benutzer bzw. Wäschestücke, so dass eine erfindungsgemäße Kontrollvorrichtung für ein Passieren des Zugangs von Wäschestücke bzw. ein Passieren von Personen durch wenigstens ein Sperrorgan gesperrt wie auch entsperrt sein kann. Dabei ist erfindungsgemäße vorgesehen, dass Wäschestücke nicht zwingend in Begleitung einer Person den Zugang passieren muss. Beispielsweise und insbesondere ist berücksichtigt, dass Wäschestücke auch in Gebinde mittels Transporthilfsmittel den Zugang passieren können.

Eine erfindungsgemäße Kontrollvorrichtung ermöglicht dadurch die Kontrolle eines Zugangs zum Wäschemagazin (in dieses hinein bzw. aus diesem heraus) für ein Passieren des Zugangs von Wäschestücken bzw. Benutzern, indem eine Zugangspassierberechtigung für den jeweiligen Benutzer auf der Grundlage der erfassten Benutzerdaten wie auch gegebenenfalls unter Berücksichtigung der für einen Benutzer aus dem Wäschemagazin entnommen bzw. maximal entnehmbare Wäschestücke festgelegt sein kann.

Die Steuerungseinrichtung kann die Ermittlung der Zugangspassierberechtigung mittels eines Datenspeichers verwalten, in dem die zur Ermittlung der Zugangspassierberechtigung eines Benutzers notwendigen Daten gespeichert sind. Dies können neben Benutzeridentifikationsdaten ebenfalls Daten für eine grundsätzliche Berechtigung zum Zugang des Wäschemagazins sein, um die Ermittlung einer Zugangspassierberechtigung im Sinne der Erfindung beschleunigen zu können. Dabei kann der Datenspeicher auch in einem unabhängig von einer erfindungsgemäßen Kontrollvorrichtung in einem weiteren System gebildet sein. Die Zugangspassierberechtigung kann dabei auf vielfäligte Art und Weise ermittelt und verwaltet werden, wie dies im Weiteren beschrieben ist.

Eine erfindungsgemäß ausgeführte Kontrollvorrichtung ist dadurch gekennzeichnet, dass die Steuerungseinrichtung erste Erfassungsmittel aufweist, die zur berührungslosen Erfassung von Wäschestückidentifikationsdaten einzelner Wäschestücke aus einer Mehrzahl in wahlfreier Anordnung zueinander und in wahlfreier Zusammenstellung den Zugang passierender Wäschestücke eingerichtet und ausgebildet sind.

Erfindungsgemäß ist durch die Möglichkeit einer wahlfreier Zusammenstellung berücksichtigt, dass verschiedenartige Wäschestücke miteinander zeitgleich von den ersten Erfassungsmitteln erfassbar sind, ohne dass die Anordnung und Zusammenstellung der Kleidungsstücke aufgegeben werden muss. Gleiches gilt für die Erfassung der im Weiteren genannten zweiten Erfassungsmittel für die Erfassung von Wäschestückidentifikationsdaten retournierter Wäschestücke. Dadurch kann der Erfassung der Wäschestückidentifikationsdaten vollautomatisch durch eine erfindungsgemäße Kontrollvorrichtung erfolgen.

Dadurch ist es möglich, dass die Wäschestücke automatisiert durch eine erfindungsgemäße Kontrollvorrichtung erfasst werden können, ohne dass die einzelnen Wäschestücke voneinander separiert und besonders ausgerichtet werden müssen, damit diese durch erste Erfassungsmittel hinsichtlich der Wäschestückidentifikationsdaten erfasste werden können.

Im Sinne der Erfindung ist unter einem Wäschemagazin ein für Personen bzw. Benutzer zugänglicher Raum zu verstehen, in dem beispielsweise und insbesondere saubere Wäschestücke aufbewahrt bzw. bevorratet sind, um Wäschestücke bzw. ein Wäschestück auf dem Wäschemagazin für dessen bestimmungsgemäßen Gebrauch entnehmen bzw. zum Zwecke des Auffüllens des Wäschemagazins bzw. zur Rückgabe von Wäschestücken in das Wäschemagazin einbringen zu können. Ferner ist es erfindungsgemäß möglich, in einem Wäschemagazin getrennt von den sauberen Wäschestücken verunreinigte Wäschestücke z.B. für Reinigungszwecke aufzubewahren.

Dabei ermöglicht es die Erfindung , dass die bauliche Ausgestaltung eines ein Wäschemagazin keinen wesentlichen Einschränkungen durch eine erfindungsgemäße Kontrollvorrichtung unterliegt.

Ferner ermöglicht erfindungsgemäß ein bidirektionale Zugang ein Passieren des Zugangs in ein Wäschemagazin wie auch aus diesem hinaus. Dadurch ist es erfindungsgemäß umfasst, dass Wäschestücke wie auch Personen über den Zugang in bzw. aus dem Wäschemagazin gelangen können. Dabei können die Wäschestücke - wie zuvor ausgeführt - zusammen mit wie auch unabhängig von einem tatsächlichen Passieren eines Benutzers den Zugang zum Wäschemagazin bzw. in dieses hinein passieren. Diese Eigenschaft bietet sich insbesondere für das Einbringen von Wäschestücken in das Wäschemagazin zum Zwecke des Auffüllens des Bestandes an.

Demzufolge ist erfindungsgemäß unter einem bidirektionalen Zugang ein Zugang zu verstehen, der als Eingang zu einem Wäschemagazin wie auch als Ausgang aus dem Wäschemagazin verwendbar ist, um durch den entsprechen eingerichteten und ausgebildeten Zugang in das Wäschemagazin zu gelangen wie auch aus diesem hinaus. Dabei bietet erfindungsgemäß ein Zugang die Zugänglichkeit zu einem Wäschemagazin für Personen wie auch Wäschestücke. Ferner umfasst erfindungsgemäß ein bidirektionalen Zugang die Möglichkeit, diesen insbesondere unidirektional nutzen zu können.

Im Weiteren ist zur einfacheren Lesbarkeit der Begriff des Wäschestücks insbesondere im Singular verwendet, wobei die entsprechenden Ausführungen sinngemäß auch für mehrere Wäschestücke gelten. Analoges gilt bei Verwendung des Begriffs im Plural. Zudem sind die zu den vorteilhaften bzw. zweckmäßigen Weiterbildungen genannten Ausführungsoptionen als beispielsweise und insbesondere Ausführungsformen einer erfindungsgemäßen Kontrollvorrichtung bzw. dessen Verwendung/Handhabung zu verstehen, selbst falls diese nicht als solche explizit bezeichnet sind.

Die Wäschestücke können dabei beispielsweise und insbesondere auch mittels eines Transportmittels in das Wäschemagazin eingebracht, wie auch aus diesem entnommen werden. Transportmittel im Sinne der Erfindung können Kartons, Kisten, Körbe und dergleichen sein, die auch zur einfacheren Handhabung vorzugsweise auf Rollen, beispielsweise mittels eines fahrbaren Gestells oder einer Sackkarre, bewegt werden können. Dabei können u.a. auch stapelbare Transportmittel verwendet werden. Die Wäschestücke können darüber hinaus gebündelt wie auch in einer Verpackung verpackt sein. Die Wäschestücke können erfindungsgemäß beliebig zusammengestellt wie auch angeordnet/ausgerichtet sein und daher von unterschiedlicher Art und Größe sein. Dabei ist erfindungsgemäß auch vorgesehen, dass lediglich ein Wäschestücke für ein Passieren des Zugangs vorgesehen ist und demgemäß entsprechend zu erfassen ist.

Die Erfindung ermöglicht es, dass die Wäschestücke in dieser Art den Zugang der Zugangseinrichtung passieren können, um von der Kontrollvorrichtung erfasst zu werden. Daher entsteht der Vorteil, dass die Wäschestücke zum Zwecke der Erfassung durch eine erfindungsgemäße Kontrollvorrichtung nicht von einander separiert und in eine besondere Ausrichtung gebracht werden müssen. Gleichfalls ist es erfindungsgemäß vorgesehen, dass ein Passieren von einzelnen Wäschestücke, die beispielsweise durch den Benutzer zum Passieren des Zugangs mitgeführt werden, von der erfindungsgemäßen Kontrollvorrichtung erfasst werden.

Erfindungsgemäß sind unter Wäschestücken jegliche Art von Textilien zu verstehen, die vorzugsweise zum mehrfachen Gebrauch vorgesehen sind und dazu zwischenzeitlich nach Gebrauch gereinigt werden. Darunter sind erfindungsgemäß beispielsweise und insbesondere Bekleidungsstücke, vorzugsweise Berufsbekleidungsstücke, zu verstehen. Ferner sind im Sinne der Erfindung von dem Begriff Wäschestücke auch Schuhe, insbesondere Berufs- bzw. Sicherheitsschuhe, umfasst.

Die Ermittlung der Zugangspassierberechtigung kann erfindungsgemäß z.B. mehrstufig erfolgen, indem zunächst auf der Grundlage der durch die Mittel zur Erfassung von Benutzeridentifikationsdaten erfassten Benutzeridentifikationsdaten die Steuerungseinrichtung eine grundsätzliche Zugangspassierberechtigung für den Benutzer (Zugangspassierberechtigung erster Stufe) ermittelt. Die weiteren Ausführungen zur Ermittlung einer Zugangspassierberechtigung für den Benutzer kennzeichnen eine beispielsweise und insbesondere Möglichkeit der Ermittlung einer Zugangspassierberechtigung.

Sofern eine Zugangspassierberechtigung erster Stufe für den Benutzer gegeben ist, erfolgt die Ermittlung einer wäschestückbasierten Zugangspassierberechtigung (Zugangspassierberechtigung zweiter Stufe) auf Basis einer festgelegten Obergrenze für die Entnahme von Wäschestücken, die dem Benutzer, also der konkret zum Wäschemagazin (bzw. aus diesem hinaus) zugangsbegehrenden Person, zugeordnet ist und durch die festgelegt ist, welche Menge und Art an Wäschestücken von der Person aus dem Wäschemagazin maximal entnehmbar ist. Dazu werden die Wäschestücke, die durch den Benutzer bereits aus dem Wäschemagazin entnommen wurden, ergänzt um die zur Entnahme aus dem Wäschemagazin vom Benutzer vorgesehenen Wäschestücke. Daraus ermittelt die Steuerungseinrichtung einer erfindungsgemäßen Kontrollvorrichtung eine Gesamtzahl von Wäschestücken für den Benutzer und vergleicht diese mit der zuvor genannten festgelegten Obergrenze.

Überschreitet die Gesamtzahl der Kleidungsstücke diese Obergrenze, ermittelt die Steuerungsvorrichtung eine negative Zugangspassierberechtigung und steuert das Sperrorgan (bzw. die Sperrorgane) der Zugangsvorrichtung derart, dass diese in einen Sperrzustand wechselt oder verbleibt (bzw. wechseln oder verbleiben), wodurch die Zugangsvorrichtung ein Passieren des Zugangs der Zugangsvorrichtung für die Person bzw. die Wäschestücke verweigert.

Sofern die Gesamtzahl der einer Person zugeordneten Wäschestücke die zuvor genannte Obergrenze unterschreitet, steuert die Steuerungseinrichtung das bzw. die Sperrorgane der Zugangseinrichtung zum Wechsel in einen Entsperrzustand, in dem der Zugang der Zugangseinrichtung für ein Passieren von Personen bzw. Wäschestücken entsperrt ist.

Dies kann beispielsweise und insbesondere auf einfachste Art und Weise mittels einer Signalanlage bzw. Anzeigeeinrichtung erfolgen, die dem Benutzer das Überschreiten der Obergrenze optisch bzw. akustisch anzeigt. Alternativ ist es möglich, dass die Zugangsvorrichtung den Zugang für ein Passieren mittels einer Schranke, einem Drehkreuz wie auch einer Tür oder ähnlichem sperrt bzw. entsperrt, so dass der Zugang nicht passierbar bzw. passierbar ist.

Erfindungsgemäß ist ein Sperrorgan vorzugsweise als steuerbare Barriere mit einem physikalischer bzw. mechanischer Widerstand derart eingerichtet und ausgebildet, dass diese gemäß der vorstehenden Ausführungen durch die Steuerungseinrichtung für eine Sperrung des Zugang bzw. Entsperrung des Zugangs steuerbar ist.

Die Personenerkennung kann beispielsweise und insbesondere dadurch erfolgen, dass die Mittel zur Erfassung von Benutzeridentifikationsdaten des Benutzers die einen Benutzer identifizierenden Merkmale erfassen, wie dies im einfachsten Fall eine Personalnummer sein kann.

Daraufhin können die Benutzeridentifikationsdaten mit Daten in Beziehung gesetzt werden, wie sie beispielsweise in einem Datenspeicher verwaltet werden und eine zum Zwecke der Ermittlung einer Zugangspassierberechtigung ausreichende Benutzeridentifikation ermöglichen.

Beispielsweise und insbesondere kann die Benutzererkennung einer erfindungsgemäßen Kontrollvorrichtung derart eingerichtet und ausgebildet sein, dass ein Benutzer eindeutig durch die Mittel zur Erfassung von Benutzeridentifikationsdaten des Benutzers identifizierbar bzw. erkennbar ist.

Auf einfache Art und Weise ist es dabei möglich, eine Benutzerkennung vorzunehmen, in dem die Mittel zur Erfassung von Benutzeridentifikationsdaten des Benutzers ein Terminal aufweisen, an die sich der Benutzer, beispielsweise mittels eines maschinenlesbaren Identifikationsmediums identifiziert. Weiter vereinfachend kann die Personenkennung auch durch Eingabe einer Identifikationsnummer (oder eines Codes für den Zugang) über ein Tastenfeld an dem Terminal erfolgen, um daraufhin durch die eingegebene Identifikationsnummer eine Benutzererkennung/- identifikation mittels der zu dem jeweiligen Benutzer in einem Datenspeicher gespeicherter Daten vorzunehmen.

Die zur Ermittlung einer Zugangspassierberechtigung für einen Benutzer notwendigen Daten können erfindungsgemäß in ihrer Art und im Umfang in weiten Grenzen gewählt werden, so dass es nicht zwingend notwendig ist, alle benutzeridentifizierenden Daten für die Ermittlung einer Zugangspassierberechtigung zu verwenden, so dass eine Benutzeridentifikationsnummer zum Zwecke der Ermittlung einer Zugangspassierberechtigung ausreichend sein kann.

Dazu kann die Steuerungseinrichtung beispielsweise und insbesondere derart eingerichtet und ausgebildet sein, dass diese auf Daten zugreift, die zu den Benutzer bzw. den/ das Wäschestück(en) in einem für die Steuerungseinrichtung zugänglichen Datenspeicher hinterlegt sind und die mit den von den Mittel zur Erfassung von Benutzeridentifikationsdaten des Benutzers bzw. den ersten Erfassungsmitteln zur berührungslosen Erfassung von Wäschestückidentifikationsdaten ermittelten Benutzeridentifikationsdaten bzw. Wäscheidentifikationsdaten in Beziehung setzbar sind.

Dadurch können die zuvor genannten Benutzeridentifikationsdaten bzw. die Wäschestückidentifikationsdaten auf einen Eintrag in einem Datenspeicher verweisen, dem die bzw. weitere Daten für die Personenerkennung bzw. Wäschestückerkennung entnehmbar sind.

Beispielsweise und insbesondere ist durch den Aufbau einer Beziehung der Benutzeridentifikationsdaten bzw. der Wäschestückidentifikationsdaten zu den in einem Datenspeicher gespeicherten Daten eine Personenerkennung bzw. Wäschestückerkennung mit einem beliebigen Detaillierungsgrad durch die Steuerungseinrichtung einer erfindungsgemäßen Kontrollvorrichtung möglich.

Vor diesem Hintergrund würde die Steuerungseinrichtung für die Ermittlung der Zugangspassierberechtigung Zugriff auf einen Datenspeicher nehmen, in dem, insbesondere wäschestück- bzw. benutzerbezogene, Daten zu vergangenen Passiervorgänge gespeichert sind, auf deren Grundlage eine Zugangspassierberechtigung für den Transfer von Wäschestücken in das Wäschemagazin bzw. aus dem Wäschemagazin ermittelbar ist.

Derartige Datenspeicher sind erfindungsgemäß beispielsweise mittels entsprechender Informationstechnologien und Datenverwaltungssysteme, z.B. Datenbanksysteme bzw. Datenmanagementsysteme, verwaltbar. Demgemäß umfasst ein erfindungesgemäßes Verwalten von Daten insbesondere ein Speichern, Lesen und Ändern und in Beziehung setzen/Verbinden von Daten des Datenspeichers.

Der Datenspeicher zur Verwaltung der Benutzeridentifikationdaten bzw. Wäschestückidentifikationsdaten kann beispielsweise und insbesondere auch Bestandteil der Steuerungseinrichtung einer erfindungsgemäßen Kontrollvorrichtung sein wie auch einem weiteren System zugeordnet sein, das der zuvor genannten Steuerungseinrichtung den mittels einer Datenanbindung Zugriff auf die darin gespeicherten Daten ermöglicht, wie dies im weiteren ausgeführt ist.

Dabei ist es möglich, dass die einer Person zugeordnete Obergrenze für von der Person maximal aus dem Wäschemagazin entnehmbare Wäschestücke, in einem der oben genannten Datenspeicher zu den Benutzeridentifikationsdaten gespeichert ist.

Ferner ist es erfindungsgemäß ebenfalls möglich, dass die Obergrenze neben weiteren für die Identifizierung eines Benutzers notwendigen Benutzeridentifikationsdaten auf einem maschinenlesbaren Personenidentifikationsmedium gespeichert ist, welches von den Mitteln zur Erfassung von Benutzeridentifikationsdaten einer erfindungsgemäßen Kontrollvorrichtung erfassbar ist, um die darauf gespeicherten Benutzeridentifikationsdaten für die Ermittlung einer Zugangspassierberechtigung durch die Steuerungseinrichtung zu verwenden.

Zudem ist es möglich, dass der Datenspeicher auf einem maschinenlesbaren Informations- bzw. Identifikationsmedium wenigstens teilweise gebildet ist. Dies kann beispielsweise unter Verwendung von mehrfach speicherungsfähigen sogenannten Chipkarten erfolgen

Der Detaillierungsgrad für die Erkennung eines Wäschestückes kann ebenfalls anforderungsspezifisch hoch oder niedrig gewählt sein. Beispielsweise und insbesondere kann für die Wäschestückerkennung eine Erkennung der Art des Wäschestückes (z.B. Oberhemd, Hose, Kittel usw.) wie auch dessen Größe, insbesondere Konfektionsgröße, zum Zwecke der Erfassung und Auswertung ausreichend sein. Ferner ist es möglich, ein Wäschestück anhand einer Nummer zu identifizieren, welche einen ausreichenden Aufschluss über das Wäschestück zu dessen Erkennung bietet. Die Anforderungen zur Identifikation eines Wäschestückes bzw. eines Benutzers können daher sehr unterschiedlich sein.

Diese Wäschestückidentifikationsdaten können beispielsweise und insbesondere in einer Identifikationsnummer oder einem Identifikationscode enthalten sein, die an dem Wäschestück, beispielsweise und insbesondere mittels eines maschinenlesbaren Wäscheidentifikationsmediums, angeordnet sind.

Im Sinne der Erfindung ist unter "maschinenlesbar" die Eigenschaft zu verstehen, dass eine manuelle Eingabe von Daten durch eine Person bzw. den Benutzer zum Zwecke der Identifikation entbehrlich ist. Maschinenlesbar sind beispielsweise und insbesondere optoelektronisch wie auch elektronisch lesbare Identifikationsmittel, wie es Magnetstreifen, Chipkarten (u.a. auch als Smartcard wie auch Integrated Circuit Card (ICC) bezeichnet), RFID-Transponder, Strich-, Punktwie Balkencodes (u.a. Barcodes, QR-Codes, ...) und dergleichen sein können. Diese Identifikationsmittel können insbesondere als Personenidentifikationsmedium dienen. Als Wäscheidentifikationsmediums kann für wahlfreien Anordnung und Zusammenstellung der Wäschestücke ggf. auch eine Kombination der zuvor genannten Technologien zum Einsatz kommen, um erfindungsgemäß eine vollautomatisierte Erfassung der Wäschestückidentifikationsdaten zu ermöglichen.

Ferner ist es im Sinne der Erfindung beispielsweise und insbesondere möglich, eine Maschinenlesbarkeit mittels einer automatisierten Bilderkennung zu realisieren, um aufgrund spezifischer optischer Ausprägungen auf einen Benutzer bzw. ein Wäschestück mit der gewünschten Detaillierung bzw. Erkennung schließen zu können. Dazu können die Mittel zur Erfassung von Benutzeridentifikationsdaten bzw. Erfassungsmittel zur Erfassung von Wäschestückidentifikationsdaten zur optischen Erfassung eingerichtet und ausgebildet sein, wobei die mittels optischer Erfassung erfassten Daten in Beziehung stehen zu den zu einem Benutzer bzw. zu einem Wäschestück in einem Datenspeicher gespeicherten Daten, um die erfassten Daten in konkretisierte Benutzeridentifikationsdaten bzw. Wäschestückidentifikationsdaten zu überführen.

Auf der Grundlage einer Zuordnung von Wäschestückidentifikationsdaten zu Benutzeridentifikationsdaten ist es möglich, Wäschestücke einem Benutzer zuzuordnen, die von diesem aus dem Wäschemagazin entnommenen bzw. in das Wäschemagazin eingebrachten wurden, so dass die Ermittlung einer Zugangspassierberechtigung zu dem Wäschemagazin auf der Grundlage erfolgen kann, inwieweit ein Benutzer ein für diesen durch eine Obergrenze bestimmtes Wäschestückkontingent erschöpft hat.

Auf der Grundlage der Zugangspassierberechtigung erfolgt die Möglichkeit des Zugangs zu einem Wäschemagazin. Dazu steuert die Steuerungseinrichtung das/die Sperrorgan(e) der Zugangsvorrichtung in Abhängigkeit der ermittelten Zugangspassierberechtigung zum Wechsel von einem Sperrzustand in einen Entsperrzustand.

Diese Zugangspassierberechtigung soll insbesondere gewährleisten, dass unberechtigten Personen ein Zutritt zum Wäschemagazin verwehrt ist. Darüber hinaus soll über die Erfassung von Wäschestückidentifikationsdaten die Wäschestücke ermittelt werden, die in ein Wäschemagazin eingebracht bzw. aus dem Wäschemagazin entnommen werden, um daraufhin auf den Bestand innerhalb des Wäschemagazins schließen zu können. Ferner kann eine erfindungsgemäße Kontrollvorrichtung dazu dienen, dass verunreinigte Wäschestücke nicht auf ungewünschte Art und Weise in das Wäschemagazin gelangen. Dies ist insbesondere bei hygiene-sensiblen Wäschemagazinen notwendig, wie sie beispielsweise bei einem Wäschemagazin für Berufsbekleidung von Pflege- wie auch Operationspersonal in einem Krankenhaus zu finden sind.

Die Zugangsvorrichtung kann dazu auf verschiedene Art und Weise ausgebildet sein. Beispielsweise und insbesondere ist es möglich, eine Tür als Sperrorgan vorzusehen, die für Zugang zum Wäschemagazin (in dieses hinein wie auch aus diesem heruas) zwischen einem Sperrzustand, indem der Zugang für ein Passieren zum Wäschemagazin gesperrt ist, und einem Entsperrzustand, in dem der Zugang für ein Passieren zum Wäschemagazin entsperrt ist, durch die Steuerungseinrichtung steuerbar ist.

Erfindungsgemäß umfasst ein Passieren des Zugangs die Möglichkeiten, in das Wäschemagazin hinein wie auch aus diesem heraus zu gelangen.

Wie bereits zuvor ausgeführt, erfolgt durch die von der Steuerungseinrichtung einer erfindungsgemäßen Kontrollvorrichtung ermittelte Zugangspassierberechtigung die Möglichkeit des Passierens des Zugangs zu einem Wäschemagazin. Dazu steuert die Steuerungseinrichtung die Zugangsvorrichtung bzw. dessen Sperrorgan(e) in Abhängigkeit der ermittelten Zugangspassierberechtigung des Benutzers zum Wechsel von einem Sperrzustand in einen Entsperrzustand.

Dazu ist - wie bereits ausgeführt - erfindungsgemäß berücksichtigt, dass die Steuerungseinrichtung die erfassten Benutzeridentifikationsdaten und die zu den Benutzeridentifikationsdaten in Beziehung gesetzten erfassten Wäschestückidentifikationsdaten in einem Datenspeicher für die Ermittlung einer Zugangspassierberechtigung verwaltet.

Dadurch entsteht der Vorteil, dass die betreffenden Daten für die Ermittlung einer Zugangspassierberechtigung eines Benutzers auf der Grundlage der durch die Mittel zur Erfassung von Benutzeridentifikationsdaten bzw. der durch die ersten Erfassungsmittel zur Erfasssung von Wäschestückidentifikationsdaten innerhalb eines Datenspeichers verwaltet werden können, der wiederum für weitere Datennutzungszwecke dienen kann. In dem Datenspeicher können beispielsweise konkrete Benutzeridentifikationsdaten wie auch konkretisierte Wäschestückidentifikationsdaten gespeichert sein, so dass eine Datenorganisation vereinfacht ist und in einem verkürzten Zeitfenster erfolgen kann. Ferner ist eserfindungsgemäß möglich, dass die in dem Datenspeicher gespeicherten Daten für weitere Auswertungs- bzw. Verwendungsmöglichkeiten nutzbar sind.

Der Datenspeicher kann dazu - wie bereist zuvor ausgeführt - der Steuerungseinrichtung einer erfindungsgemäßen Kontrollvorrichtung zugehörig sein wie auch für den Zugriff durch diese Steuerungseinrichtung in einem von der erfindungsgemäßen Kontrollvorrichtung unabhängigen System bereitgestellt sein. Der Zugriff auf den Datenspeicher kann erfindungsgemäß beispielsweise und insbesondere datenschreibend/-verändernd wie auch datenlesend eingerichtet sein.

Demgemäß kann eine Konkretisierung von Benutzeridentifikationsdaten wie auch lNäschestückidentifikationsdaten auf der Grundlage der in dem Datenspeicher gespeicherten Daten erfolgen. Erfindungsgemäß ist es möglich, dass der Datenspeicher auch Teil eines Warenwirtschafts- oder eines übergeordneten Datensystems ist, und damit zur Nutzung durch verschiedene Anwendungen bereit steht. Zudem ist die Datenhandhabung, beispielsweise zur Aktualisierung, vereinfacht. Zur Verwaltung der betreffenden Daten in einem Datenspeicher bieten sich vielfältige Möglichkeiten, insbesondere unter Verwendung von Netzwerktechnologien (z.B. Internettechologien).

Ein Datenspeicher kann physikalisch beispielsweise durch eine Festplatte wie auch einen Speicherchip gebildet sein. Desweiteren eignen sich insbesondere jeglich Art von mehrfach beschreibbaren Datenmedien, die eine Datenspeicherung ermöglichen, wie es u.a. auch optische Speichermedien sein können.

Im Sinne der Erfindung ist es ebenfalls vorgesehen, dass ein Wäschestück ein maschinenlesbares Wäscheidentifikationsmedium aufweist, kraft dessen ein Wäschestück durch eine Leseeinheit für das Informationsmedium identifiziert werden kann. Dies kann beispielsweise dadurch erfolgen, dass an dem Wäschestück ein so genannter Barcode angeordnet ist, der mittels einer Leseeinheit, die insbesondere als optische Leseeinheit (Scanner) ausgebildet ist, den an dem Wäschestück angebrachten Barcode auszulesen vermag. Dazu kann eine entsprechend Farbmarkierung an dem Kleidungsstück zur Ausbildung eines Barcodes dienen, der an unterschiedlichen Stellen angeordnet ist. Der Barcode kann beispielsweise auch mittels Magnetstreifen gebildet sein. Ferner ist es möglich, dass ein Wäschestück zur Identifizierung ein für das menschliche Auge im nicht sichtbaren Bereich maschinenauslesbares Muster aufweist, dass z.B. mittels Ultraschall oder Röntgenstrahlung auslesbar ist.

Dazu ermöglicht die Erfindung den Vorteil einer Überwachung der entnommenen Kleidungsstücke, wodurch der tatsächliche Bedarf an Wäschestücken für eine Person oder eine Personengruppe ermittelt werden kann. Ferner ermöglicht es die Erfindung, die einem Wäschemagazin entnommenen Wäschestücke hinsichtlich ihres Verbleibs beispielsweise und insbesondere bei einer Person/ eines Benutzers kontrollieren zu können.

Eine vorteilhafte Weiterbildung einer erfindungsgemäßen Kontrollvorrichtung sieht vor, dass die Steuerungseinrichtung zweite Erfassungsmittel aufweist, die für eine berührungslose Erfassung der Wäschestückidentifikationsdaten von über eine Rückgabeeinrichtung retournierter Wäschestücke eingerichtet und ausgebildet ist, wobei die Steuerungseinrichtung die Wäschestückidentifikationsdaten der retournierten Wäschestücke den Benutzeridentifikationsdaten des Benutzers zuordnet und diese Zuordnung in dem Datenspeicher verwaltet.

Dadurch ergibt sich der Vorteil, dass ein Wäschestück, z.B. nach dessen Gebrauch zum Zwecke einer Reinigung, nicht zwingend dem Wäschemagazin zurückgeführt werden muss, sondern beispielsweise an einer entfernt von dem Wäschemagazin für die Rückgabe mittels einer Rückgabeeinrichtung vorgesehenen Stelle vorgenommen werden kann.

Für die zweiten Erfassungsmittel können sich ebenfalls die zuvor genannten Merkmale der ersten Erfassungsmittel ergeben, so dass der Einfachheit halber an dieser Stelle ein entsprechender Verweis auf die zuvor genannten Ausführungen bzw. die nachfolgenden Ausführungen zu den ersten Erfassungsmitteln genügen soll, die entsprechend analog auch auf die zweiten Erfassungsmittel anwendbar sind.

Erfindungsgemäß ist es berücksichtigt, dass ein Benutzer eine maximale Anzahl von Wäschestücken einem Wäschemagazin entnehmen darf. Das entsprechende Kontingent an Wäschestücken für einen Benutzer ist durch eine Obergrenze bestimmt, wie diese bereits eingangs gekennzeichnet wurde.

Im Rahmen der Erfindung ist es vorgesehen, das die von einem Benutzer dem Wäschemagazin entnommenen Wäschestücke den Benutzeridentifikationsdaten zugeordnet sind. Die Wäschestücke werden durch Gebrauch verunreinigt, so dass ein durch bestimmungsgemäßen Gebrauch verunreinigtes Wäschestück zum Zwecke dessen Reinigung zuzuführen ist. Dadurch wird ein solches durch die betreffende Person retourniert. Damit steht es der Person zum Gebrauch nicht mehr zur Verfügung, so dass diese ein gereinigtes bzw. sauberes Wäschestück dem Wäschemagazin entnehmen darf. Daher ist die Anzahl der von einem Benutzer dem Wäschemagazin entnommenen Wäschestücke, wie sie als Benutzerdatum in dem Datenspeicher gespeichert ist, durch die Anzahl der für sie bzw. von ihr retournierten Wäschestücke zu korrigieren bzw. herabzusetzen.

Für eine Automatisierung ist erfindungsgemäß vorgesehen, dass eine Erfassung von Wäschestückidentifikationsdaten für die Erfassung der für z. B. die Reinigung, insbesondere außerhalb des Wäschemagazins bzw. des Zugangs zum Wäschemagazin, retournierter Wäschestücke erfolgen kann.

Eine Retour von Wäschestücken z.B. zum Zwecke deren Reinigung kann auf verschiedenste Art und Weise erfolgen. Beispielsweise und insbesondere kann die Retour eines Kleidungsstückes innerhalb des Kleidermagazins erfolgen, so dass eine Person für die Retour des Kleidungsstücke in dem Wäschemagazin den Zugang der Zugangseinrichtung unter Berücksichtigung der Zugangspassierberechtigung passiert und das betreffende Kleidungsstück einem für die Retour des Kleidungsstückes vorgesehenen Bereich zuführt. Beim Passieren des Zugang in Richtung in das Wäschemagazins erfassen die ersten Erfassungsmittel die Wäschestückidentifikationsdaten das betreffende Wäschestücke, während dieses beim Verlassen des Wäschemagazins von der betreffenden Person nicht mitgeführt wird. Dadurch erkennt das Steuerungseinrichtung, dass das entsprechende Wäschestück retourniert wurde, woraufhin die Anzahl der von der Person aus dem Wäschemagazin entnommenen Wäschestücke im Datenspeicher der Steuerungseinrichtung entsprechend korrigiert wird. Dazu werden die retournierten Wäschestücke erfindungsgemäß insbesondere in dem zuvor genannten Datenspeicher verwaltet.

Ferner ist es erfindungsgemäß beispielsweise und insbesondere möglich, eine Retour für die Reinigung eines Wäschestückes außerhalb des Wäschemagazins wie auch vorzugsweise außerhalb der Zugangs vorzusehen. Dadurch wird auf einfache Art und Weise erreicht, dass ein hygiene-sensitives Wäschemagazin nicht unnötig mit Verunreinigung durch verunreinigte Wäschestücke kontaminiert wird. Demzufolge sind die Mittel zur Erfassung von Wäschedaten derart einzurichten und auszubilden, dass eine Erfassung der Wäschestücke auch außerhalb des Zugangs bzw. des Wäschemagazins erfolgen kann. Auf einfache Art und Weise kann dies dadurch automatisiert werden, in dem die Erfassung der retournierten Wäschestückidentifikationsdaten unter Verwendung eines maschinenauslesbaren Identifikationsmedium vorgenommen wird, wie diese zuvor beschrieben wurden. Dazu kann beispielsweise eine Leseeinheit für die Erfassung eines an einem Wäschestück angeordneten RFID-Transponders an dem für die Retoure des Wäschestückes vorgesehenen Bereich angeordnet sein.

Im Hinblick auf die erfindungsgemäße automatisierte Erfassung von maschinenlesbaren Wäschestückidentifikationsdaten ist es möglich, über so genannte Bilderkennungsmittel eine Erkennung von Wäschestücken vorzunehmen. Dazu könnte beispielsweise eine optoelektronische Leseeinheit (u.a. auch als Scanner bezeichnet) vorzugsweise ein Wäschestück unter anderem mittels dessen Kontur erfassen, so dass die Steuerungseinrichtung einer erfindungsgemäßen Kontrollvorrichtung daraufhin auf das Wäschestück rückzuschließen vermag. Ferner könnten eine Kennzeichnung auf der Oberfläche des Wäschestückes, die lediglich beispielsweise durch eine Lichtstrahlung in einem bestimmten Frequenzbereich erkennbar ist, zur Erfassung der betreffenden Wäschestückidentifikationsdaten beitragen.

Ferner ist es möglich, optische Codes, wie es so genannte Punkt-, Strich- bzw. Balkencodes (z.B. Barcodes, QR-Codes) sein können, zu verwenden, um diese an einem Wäschestück allseitig erfassbar anzuordnen, woraufhin durch eines beispielsweise Barcodes-Leseeinheit als Bestandteil der Mittel zur Erfassung von Wäschestückidentifikationsdaten eine Identifizierung eines Wäschestückes durch die Steuerungseinrichtung ermöglicht ist.

Zudem ist es erfindungsgemäß möglich, dass das Gewebe des Wäschestückes entsprechend zur Identifikation des Wäschestückes ausgebildet ist. Dies könnte beispielsweise durch Einbringen von Metallfäden z.B. nach einem bestimmten Muster als Informationsmedium erfolgen, die mittels elektromagnetischer Strahlung einer Identifikation des Wäschestückes ermöglichen.

Die Verwendung derartiger Wäschestückidentifikationsmedien führt dazu, dass eine quasi eindeutige Identifizierung eines Wäschestückes automatisiert durch eine erfindungsgemäße Kontrollvorrichtung ermöglicht ist, so dass die Erfassung dieses Wäschestückes zur Kontrolle des Transfers aus bzw. in das Wäschemagazin vereinfacht ist.

Dazu ist in einer weiteren vorteilhaften Weiterbildung der Erfindung vorgesehen, dass die ersten Erfassungsmittel bzw. die zweiten Erfassungsmittel (jeweils) wenigstens eine Leseeinheit zur Erfassung von mittels elektromagnetischer Strahlung übertragener Wäschestückidentifikationsdaten aufweisen, die auf einem an dem Wäschestück angeordneten maschinenauslesbaren Informationsmedium gespeicherten sind. Dabei ist erfindungsgemäß das Informationsmedium insbesondere ein RFID-Transponder, vorzugsweise ein Ultrahochfrequenz-RFID-Transponder, so dass die Leseeinheit dementsprechend zur Erfassung der auf dem RFID-Transponder gespeicherten Wäschestückidentifikationsdaten eingerichtet und ausgebildet ist und insbesondere eine RFID-Leseeinheit bzw. RFID-Leser ist.

Dadurch entsteht der Vorteil, dass eine automatisierte und berührungslose Erfassung von Wäschestückidentifikationsdaten auf einfache wie auch kostengünstige Art und Weise ermöglicht ist.

Zur weiteren Vereinfachung ist es möglich, eine automatisierte Erkennung eines Wäschestückes bzw. Erfassung von Wäschestückidentifikationsdaten unter Verwendung von RFID-Technologien im Bereich eines Wäschelagerraumes einzusetzen, wie dies erfindungsgemäß dadurch berücksichtigt ist, dass die Mittel zur Erfassung von Wäschestückidentifikationsdaten wenigstens einer RFID-Leseeinheit aufweisen zum Auslesen eines an einem Wäschestück angeordneten RFID-Transponders, insbesondere eines Ultrahochfrequenz-RFID-Transponders.

Die Verwendung von RFID-Technologien eröffnet die Möglichkeit, Wäschestücke auf einfache Art und Weise erfassen und hinsichtlich einer Logistikdokumentation von Wäschestückidentifikationsdaten handhaben zu können. RFID-Technologien basieren grundsätzlich auf der Verwendung einer Leseeinheit und eines sogenannten RFID-Transponders, wobei die Leseeinheit die Informationen des RFID-Transponser erkennt und ausliest.

RFID-Technologien sind beispielsweise in Kaufhäusern zur Diebstahlprävention verbreitet, wobei eine Empfangseinrichtung einer Diebstahlanlage das unberechtigte Passieren eines Bereiches anhand eines mittels an der Ware angebrachten RFID-Transponsers erkennt. Dabei erfolgt ein Signal zur vermeidlichen Diebstahlanzeige eines nicht von der Ware entfernten oder auf andere Weise deaktivierten RFID-Transponders. Der RFID-Transponser dient dabei lediglich als Indikator der Warenzugehörigkeit zu dem Kaufhaus und nicht der Erkennung/Identifikation/Eingrenzung einer Ware.

RFID-Transponder, die im Weiteren auch verkürzt als RFID-Tags bezeichnet werden, gibt es sowohl in der Ausführung als aktives Identifikationsmittel wie auch als passives Identifikationsmittel.

Die Verwendung eines aktiven Identifikationsmittels bedingt, dass die notwendige Energie dem RFID-Tag zugeführt wird, so dass beispielsweise und insbesondere ein batteriebasierter Betrieb eines aktiven RFID-Tags vorgesehen ist, kraft dessen der RFID-Tag Informationen aktiv aussendet, die daraufhin von einer Leseeinheit erfasst und für die weitere Bearbeitung der erfassten Daten weitergeleitet werden können.

Aktive RFID-Tags haben den Vorzug, dass diese die gespeicherten Informationen über vergleichsweise große Distanzen übersenden können, so dass die Leseeinheit beispielsweise diese ausgestrahlten Informationen über vergleichsweise große Distanzen empfangen kann.

Darüber hinaus ist es möglich passive RFID-Tags einzusetzen, die darauf basieren, dass die in ihnen hinterlegten Informationen über vergleichsweise kurze Distanzen ausgelesen werden können. Dazu ist eine entsprechende Leseeinheit derart eingerichtet und auch ausgebildet, dass die bei dem passiven RFID-Tag hinterlegten Informationen ausgelesen werden können.

Systeme die auf der RFID-Technologie basieren, sind unter anderem von der Norm ISO 18000-1FF. genormt. Die RFID-Systeme, die gemäß dieser Norm eingerichtet und ausgestattet sind, arbeiten nach folgendem grundlegendem Schema:

Die RFID-Leseeinheit (Reader) erzeugt ein hochfrequentes elektromagnetisches Wechselfeld, dass den RFID-Transponder (RFID-Tag) erreicht. Dieses hochfrequente elektromagnetische Feld dient dazu, dem RFID -Tag Energie zuzuführen, um ein Lesen der im RFID-Tag hinterlegten Informationen zu ermöglichen.

Die Leseeinheit erhält diese Informationen, die gegebenenfalls kodiert hinterlegt sind und die zur weiteren Verarbeitung an entsprechende Bestandteile einer Steuerungseinrichtung weitergeleitet werden.

Informationen, die auf dem RFID-Tag hinterlegt sein können, sind beispielsweise eine Seriennummer, die den RFID-Tag identifiziert und die unveränderlich auf diesem hinterlegt ist. Diese kann erfindungsgemäß dazu genutzt werden, ein Wäschestück zu erkennen bzw. zu identifizieren. Auf diese Art und Weise ist es ebenfalls möglich, einen Zugangsausweis mittels eines RFID-Tags als Identifikationsmedium für einen Benutzer zu realisieren, so dass diese Technologie in analoger Weise zur Wäschestückidentifizierung bzw. - erkennung ebenfalls zur Benutzererkennung/-identifizierung nutzbar sind.

Darüber hinaus ist es möglich weitere Daten des zu kennzeichnenden Wäschestückes auf dem RFID-Tag zu hinterlegen. Zudem ist es möglich, die Seriennummer des RFID-Tags zu verwenden, um eine Identifizierung eines Wäschestückes vorzunehmen.

RFID-Tags arbeiten in unterschiedlichen Frequenzbereichen, so dass die entsprechende Frequenz mit der des RFID-System arbeitet, hersteller- wie auch länderspezifisch sein kann. Dabei wird grundsätzlich zwischen Systemen unterschieden, die im langwelligen Bereich arbeiten oder im Bereich der Ultrahochfrequenz (Ultrahochfrequenz-RFID-Transponder).

RFID-Tags, die auf Basis der Ultrahochfrequenz einen Informationstransfer ermöglichen, bieten den Vorteil, dass eine schnellere Datenübertragung ermöglicht ist. Dadurch ist es möglich, in einem vergleichsweise kurzen Zeitraum Informationen aus dem RFID-Tag auslesen zu können. Darüber hinaus ermöglichen derartige RFID-Tags die Hinterlegung weiterer Informationen, die entsprechend ausgelesen werden können.

In dem der Erfindung zugrundeliegenden Anwendungsbereich ist jedoch sicherzustellen, dass eine Reinigung der Wäsche den RFID-Tag nicht zerstört. Daher sind erfindungsgemäß entsprechend reinigungsresistente RFID-Tags zur Verwendung einer erfindungsgemäßen Kontrollvorrichtung berücksichtigt, die an einem Wäschestück angebracht werden. Dazu können derartige RFID-Tags beispielsweise bei dem betreffenden Wäschestück eingenäht sein. Ferner ist es im Sinne der Erfindung möglich, entsprechende RFID-Tags auf das betreffende Wäschestück mittels eines Klebstoffes aufzubringen. Diesbezüglich existieren weitreichende Möglichkeiten, den RFID-Tag an der Wäsche - auch abnehmbar - anzuordnen.

Im Rahmen der Erfindung ist unter Reinigung jeglicher Art der Reinigung zu verstehen, um die Wäschestücke zu reinigen. Insbesondere die Einwirkung von Wasser, Chemikalien wie auch von Hitze zur Reinigung der Wäsche stellt an den RFID-Tag wie auch dessen möglichst feste Anordnung an ein Wäschestück erhöhte Anforderungen, damit dieser RFID-Tag trotz mehrfacher Reinigung seine Funktionsfähigkeit nicht verliert.

Ferner ist es im Sinne der Erfindung möglich, eine Entnahme bzw. Austauschbarkeit des RFID-Tags an dem Wäschestück vorzusehen, um diesen vor Reinigung des Wäschestückes bzw. der Wäsche von dem betreffenden Wäschestück zu entfernen und ggf. anschließend wieder anbringen zu können. Darüber hinaus ist es dadurch möglich, den RFID-Tag vor der Reinigung zu entnehmen und nach der Reinigung des Wäschestückes durch gegebenenfalls einen anderen RFID-Tag zu ersetzen, so dass nach der Reinigung der RFID-Tag an der Wäsche angeordnet wird und zur Wäschestückerkennung eingerichtet wird.

In einer weiteren vorteilhaften Weiterbildung der Erfindung ist vorgesehen, dass die ersten Erfassungsmittel zur Erfassung von Benutzeridentifikationsdaten bzw. die Mittel zur Erfassung von Wäschestückidentifikationsdaten derart eingerichtet und ausgebildet sind, dass in Verwendungsposition der Zugangsvorrichtung eine Erfassung der Wäscheidentifikationsdaten von Wäschestücken für den Transfer aus bzw. in das Wäschemagazin bzw. eine Erfassung der Benutzeridentifikationsdaten des Benutzers zwischen dem dem Wäschemagazin zugewandten Sperrorgan und dem dem Wäschemagazin abgewandte Sperrorgan erfolgen.

Dadurch entsteht der Vorteil, dass der Erfassungsbereich der ersten Erfassungsmittel zur Erfassung von Wäschestückidentifikationsdaten auf einen begrenzten Raum konzentriert ist, wodurch eine Erfassung der zuvor genannten Daten entsprechend zielgerichtet sowie schnell und sicher erfolgen kann. Störquellen, die einer sichere Erfassung von Wäschestückidentifikationsdaten entgegenstehen, sind damit mindestens auf ein Minimum reduziert.

Grundsätzlich ist es dadurch möglich, die Erfassung von Benutzeridentifikationsdaten innerhalb des Schleusenraumes vornehmen zu können. Dies kann beispielsweise dadurch erfolgen, dass die betreffenden Benutzeridentifikationsdaten z.B. ebenfalls auf einem RFID-Transponder oder dergleichen, wie dieser zuvor beschrieben wurde, gespeichert sind, mittels der die Erfassung der Benutzeridentifikationsdaten durch die Mittel zur Erfassung von Benutzeridentifikationsdaten einer erfindungsgemäßen Kontrollvorrichtung vereinfacht werden kann.

Die Erkennung von Wäschestücken durch Erfassung ihrer Wäscheidentifikationsdaten und eine Zuordnung der durch den Zugang zum Wäschemagazin transferierten Wäschestücke zu einem Benutzer kann dadurch beschleunigt und präzisiert werden, dass der Zugang zum Wäschemagazin derart eingerichtet und ausgebildet ist, dass dieser zeitgleich lediglich durch einen einzelnen Benutzer passiert werden kann bzw. die den Zugang passierenden Wäschestück entsprechend für diesen einen Benutzer erfasst werden.

Dazu ist in einer weiteren vorteilhaften Weiterbildung der Erfindung vorgesehen, dass die Zugangseinrichtung zur wenigstens abschnittsweisen Bildung des Zugangs wenigstens eine Vereinzelungsvorrichtung aufweist, bei der insbesondere wenigstens ein Sperrorgan für eine Separation der Benutzer für ein Passieren des Zugangs eingerichtet und ausgebildet ist.

Derartige Vereinzelungseinrichtungen werden unter anderem in anderen Anwendungsbereichen auch unter der Bezeichnung "Vereinzelungsanlagen" geführt und beispielsweise in einer Ausbildung als Drehsperren realisiert, wie sie verbreitet in Kaufhäuser eingesetzt werden, um den Besucherstrom lenken zu können.

Dazu können Drehsperren aufgeteilt sein in Bereiche, die vorzugsweise zum zeitgleichen Passieren der Drehsperre lediglich von einem Benutzer genutzt werden können, um Zugang zu einem Wäschemagazin zu erhalten. Durch Nutzung einer Drehsperre werden die Benutzer zur ein Passieren des Zugangs voneinander separiert, so dass der Zugang zu dem Wäschemagazin besser kontrollierbar ist. Derartige Drehsperren können daher für den Zugang zu einem personenbegehbaren Wäschemagazin genutzt werden, in dem diese durch eine Steuerungseinrichtung hinsichtlich ihrer Passierbarkeit - wie zuvor bereits ausgeführt - gesteuert sind.

Demgemäß es möglich, derartige Drehsperren derart einzurichten und auszubilden, dass neben einer Person ebenfalls Wäschestück (auch unabhängig vom tatsächlichen Passieren eines Benutzers) über eine entsprechende Drehsperre als Beispiel einer Vereinzelungseinrichtung in das Wäschemagazin eingebracht bzw. aus diesem entnommen werden können.

Darüber hinaus ist es möglich, dass eine Vereinzelungseinrichtung ausgebildet ist als sogenanntes Drehkreuz, über das Benutzer eines Benutzerstroms (Ansammlung von Benutzers) voneinander separiert werden in einzelne Benutzer, die in einen für den Benutzer vorgesehenen Bereich des Drehkreuzes eintreten können und schließlich über eine Drehbewegung des Drehkreuzes Zugang zu dem betreffenden Wäschemagazin erhalten.

Dazu kann wiederum ein entsprechendes Drehkreuz erfindungsgemäß derart eingerichtet und ausgebildet, dass neben einem Benutzer auch Wäschestücke Zugang zu dem Wäschemagazin erhalten können. Eine Steuerung des Drehkreuzes durch die Steuerungsmittel ist ebenfalls auf einfache Art und Weise möglich.

Eine Benutzererkennung bzw. Wäschestückerkennung kann unter anderem in einer der zuvor aufgeführten Art und Weise erfolgen, so dass während des Passierens der Vereinzelungseinrichtung eine Erfassung von Wäschestückidentifikationsdaten bzw. Benutzeridentifikationsdaten erfolgen kann.

Erfindungsgemäß kann eine Vereinzelungseinrichtung auf verschiedene Art und Weise ausgebildet und eingerichtet sein, um den Transfer von Wäschestücken auf bzw. in ein Wäschemagazin besser kontrollieren zu können.

Dies kann erfindungsgemäß beispielsweise auch dadurch erfolgen, dass die Vereinzelungseinrichtung nach Art einer Personenschleuse realisierbar ist. Daher ist in einer weiteren vorteilhaften Weiterbildung der Erfindung berücksichtigt, dass die Vereinzelungseinrichtung nach Art einer Personenschleuse eingerichtet und ausgebildet ist, die für den Zugang zum Wäschemagazin in Verwendungsposition der Personenschleuse einen zwischen einem dem Wäschemagazin zugewandten ersten Sperrorgan und einen dem Wäschemagazin abgewandten zweiten Sperrorgan gebildeten Schleusenraum aufweist, wobei die Sperrorgane insbesondere für die Erfassung der Wäschestückidentifikationsdaten bzw. die Erfassung der Benutzeridentifikationsdaten durch die Steuerungsmittel, insbesondere unabhängig voneinander, zwischen einem Sperrzustand und einem Entsperrzustand steuerbar sind.

Die Art der Realisierung einer Vereinzelungseinrichtung nach Art einer Personenschleuse wird im nachfolgenden auch verkürzt mit dem Begriff der Personenschleuse gekennzeichnet. Zur Bildung einer Personenschleuse können erfindungsgemäß auch verschiedene Realisierungsmöglichkeiten einer Vereinzelungseinrichtung miteinander kombiniert werden zu einer weiteren Vereinzelungseinrichtung bzw. zur Gestaltung einer Personenschleuse.

Dabei ist es möglich, dass das erste und zweite Sperrorgan auf gleiche Art und Weise realisiert sind. Ferner ist es möglich, dass das erste gegenüber dem zweiten Sperrorgan unterschiedlich ausgebildet und eingerichtet ist. Dabei kann ein Sperrorgan beispielsweise und insbesondere auch nach Art eines Drehkreuzes bzw. einer Drehsperre eingerichtet und ausgebildet sein, die in einer Personenschleuse zum Einsatz kommt.

Dadurch entsteht der Vorteil, dass eine Kontrolle des Zugangs zu dem Wäschemagazin wie auch eine Kontrolle der Zufuhr bzw. Entnahme der Wäschestücke aus dem Wäschemagazin bzw. in das Wäschemagazin zuverlässig kontrolliert werden kann.

Für ein Sperren bzw. Entsperren des Zugang der Zugangseinrichtung einer erfindungsgemäßen Kontrollvorrichtung können als Sperrorgane beispielsweise und insbesondere steuerbare Türen, wie es u.a. Schiebe-, Falt- bzw. Schwenktüren sind, vorgesehen sein, die einen steuerbaren Zugang zu einem Wäschemagazin ermöglichen.

Dabei sind die zuvor genannten Sperrorgane in Passierrichtung, in der die Personenschleuse erfindungsgemäß durch den Benutzer bzw. das/die Waschestück(e) passierbar ist, derart beabstandet zueinander angeordnet, dass in Zusammenwirkung zwischen ihnen angeordneten seitlichen Wandungen ein Schleusenraum ausgebildet ist, der insbesondere derart eingerichtet und bemessen ist, dass zeitgleich lediglich ein Benutzer, beispielsweise und insbesondere unter Mitführung von Wäschestücken/ eines Wäschestückes, in den Schleusenraum gelangt. Dies kann auch durch eine angepasste Steuerung der Sperrorgane erreicht werden, die derart von der Steuerungseinrichtung bezüglich eines Sperrzustandes bzw. Entsperrzustandes steuerbar sind, dass diese lediglich einer Person Zutritt zum Schleusenraum gewähren.

Dadurch erwächst der Vorteil einer doppelten Sicherung des Wäschemagazins, in dem die Sperrorgane unabhängig von einander durch die Steuerungseinrichtung sperrbar bzw. entsperrbar ist. Ferner wird es möglich, dass ein Passieren des Zugangs weg- bzw. zeit- abschnittsweise steuerbar ist, indem zunächst eine dem Wäschemagazin abgewandte Zugangssperre für den Zugang in das Wäschemagazin entsperrt wird, so dass ein Benutzer bzw. Wäschestücke in einen Schleusenraum eingebracht werden können.

Die Erfassung von Benutzeridentifikationsdaten kann auf unterschiedliche Art une Weise erfolgen. In einer weiteren vorteilhaften Weiterbildung der Erfindung ist vorgesehen, dass die Mittel zur Erfassung von Benutzeridentifikationsdaten wenigstens eine Datenerfassungseinheit für das Erfassen eines maschinenauslesbaren Identifikationsmediums aufweisen, auf dem die Benutzeridentifikationsdaten gespeichert sind. Dabei können beispielsweise sogenannte Chipkarten verwendet werden, die der Benutzer mit sich führt und über eine Datenerfassungseinheit zur Erkennung für die Ermittlung einer Zugangspassierberechtigung für ein Passieren des Zugangs wie auch zur Zuordnung der betreffenden Wäschestückidentifikationsdaten zu seinen Benutzeridentifikationsdaten erfassen lässt. Beispielsweise und insbesondere kann eine Datenerfassungseinheit erfindungsgemäß auch nach Art eines Terminals eingerichtet und ausgebildet sein, um die Benutzeridentifikationsdaten erfassen zu können. Dazu bieten sich weitreichende Möglichkeiten der Realisierung einer Datenerfassungseinheit.

Beispielsweise und insbesondere kann die Schleusenkammer einer erfindungsgemäßen Kontrollvorrichtung durch die Steuerungseinrichtung wie folgt gesteuert sein:

Die Zugangseinrichtung ist zunächst in einem Sperrzustand, bei dem zunächst die Sperrorgane den Zugang zum Wäschemagazin bzw. aus diesem hinaus sperren.

Zu dem Benutzer werden zunächst Benutzerdaten durch die Mittel zur Erfassung von Benutzeridentifikationsdaten erfasst. Daraufhin wird die zuvor beschriebene Zugangspassierberechtigung erster Stufe durch die Steuerungseinrichtung ermittelt. Sofern eine Zugangspassierberechtigung erster Stufe vorliegt, wird das in Verwendungsposition dem Wäschemagazin abgewandte Sperrorgan für den Zutritt in den Schleusenraum entsperrt, wobei das dem Wäschemagazin zugewandte Sperrorgan für ein Passieren weiterhin in einem Sperrzustand verbleibt.

Nach Zutritt des Benutzers zum Schleusenraum bzw. Einbringung der Wäschestücke in den Schleusenraum erfolgt eine Erfassung von Wäschestückidentifikationsdaten durch die ersten Erfassungsmittel zur Erfassung von Wäschestückidentifikationsdaten.

Sofern Wäschestücke durch den Benutzer in den Schleusenraum eingebracht sind, die jedoch nicht in das Wäschemagazin gelangen dürfen, bleibt das dem Wäschemagazin zugewandte Sperrorgan in einem Sperrzustand, so dass der Zugang für ein Passieren weiterhin gesperrt ist, jedoch eine Zugänglichkeit zum Schleusenraum ermöglicht ist.

Das dem Wäschemagazin zugewandte Sperrorgan wird durch die Steuerungsmitteln in einen Entsperrzustand gesteuert, sobald der Benutzer keine der betreffenden Wäschestücke im Schleusenraum mitführt, die dem Wäschemagazin nicht zugeführt bzw. diesem nicht entnommen werden dürfen.

Daraufhin wird das dem Wäschemagazin zugewandte Sperrorgan durch die Steuerungseinrichtung in einen Entsperrzustand gesteuert, woraufhin der Zugang passierbar ist. Dabei ist es beispielsweise und insbesondere möglich, dass das dem Wäschemagazin abgewandte Sperrorgan in einem Sperrzustand gesteuert ist, um weiteren Personen ein zeitgleiches Passieren des Zugangs zu verweigern.

Durch die unabhängig voneinander ansteuerbaren Sperrorgane ist es möglich, den Schleusenraum für den Benutzer bzw. die Wäschestücke zunächst zugänglich zu machen, ohne dass der Zugang passierbar ist. Darauf hin kann nach erfolgter Erfassung der Wäschestückidentifikationsdaten bzw. Benutzeridentifikationsdaten ein Passieren des Zugangs ermöglicht werden. Vor diesem Hintergrund ist erfindungsgemäß berücksichtigt, die Zugänglichkeit zum Schleusenraum durch eine entsprechende Ansteuerung der Sperrorgane zu ermöglicht, ohne dass der Zugang passierbar ist. Das Passieren des Zugangs bzw. des Schleusenraumes erfolgt dazu erfindungsgemäß durch Einlass in den Schleusenraum über eines der Sperrorgane der Personenschleuse und Verlassen des Schleusenraumes über das verbleibende Sperrorgan.

Durch die Personenschleuse ist ein bidirektionaler Zugang zu einem Wäschemagazin realisiert, wobei erfindungsgemäß ebenfalls berücksichtigt ist, dass die Zugangsvorrichtung bzw. die Personenschleuse eine Vorzugsrichtung aufweist, in der diese für den überwiegenden Anteil der Erfassungs- bzw. Passiervorgänge benutzt wird. Zur Führung der Benutzer durch das Wäschemagazin ist es daher erfindungsgemäß möglich, die Personenschleuse bzw. die Zugangsvorrichtung für einen unidirektionalen Zugang in die Wäschekammer bzw. aus diese hinaus zu verwenden.

Dazu ist erfindungsgemäß vorgesehen, dass eine erfindungsgemäße Kontrollvorrichtung weitere Zugangseinrichtungen aufweisen kann. Dabei ist es möglich, dass eine der Zugangsvorrichtungen derart eingerichtet und ausgebildet ist, dass diese als Eingang zu dem Wäschemagazin und eine weitere Zugangseinrichtung als Ausgang aus dem Wäschemagazin verwendet wird. Die Zugangseinrichtungen können dabei erfindungsgemäß in ihrem Funktionsumfang unterschiedlich eingerichtet und ausgebildet sein. Ferner ist möglich, dass eine der Zugangseinrichtungen lediglich eine Zugangspassierberechtigung erster Stufe des Benutzers prüft und keine Erfassung von Wäschestückidentifikationsdaten vornimmt. Daher ist es erfindungsgemäß möglich, dass die Zugangseinrichtung zueinander unterschiedlich eingerichtet und ausgestaltet sind und zum in verschiedenen Betriebsmodi zur Kontrolle des Transfers von Wäschestücken aus bzw. in das Wäschemagazin verwendet werden.

Das dem Wäschemagazin zugewandte Sperrorgan ist zunächst für eine Zugänglichkeit des Schleusenraums entsperrt, wobei das dem Wäschemagazin abgewandte Sperrorgan in einem Sperrzustand ist. Der Benutzer tritt über das erstgenannte Sperrorgan in den Schleusenraum ein, woraufhin dieses Sperrorgan durch die Steuerungseinrichtung für ein Sperren des Passieren des Zugangs gesteuert wird.

Daraufhin werden die Wäschestückidentifikationsdaten der von dem Benutzer mitgeführten Wäschestücke von den/dem ersten Erfassungsmittel zur Erfassung der Wäschestückidentifikationsdaten erfasst. Daraufhin erfolgt die Ermittlung der Zugangspassierberechtigung wie bereits oben ausgeführt.

Sofern der Benutzer zur Entnahme der mitgeführten Wäschestücke aus dem Wäschemagazin berechtigt ist, wird die dem Wäschemagazin abgewandte Zugangssperre entsperrt, woraufhin die Person die Personenschleuse mit den mitgeführten Wäschestücken verlassen kann. Daraufhin sperrt die Steuerungseinrichtung insbesondere die dem Wäschemagazin abgewandten Zugangssperren, vorzugsweise beide Zugangssperren und prüfen die ersten Erfassungsmittel zur Erfassung von Wäschestückidentifikationsdaten, ob der Schleusenraum frei von Wäschestücken ist. Andernfalls erfolgt eine Mitteilung an eine für die Kontrollvorrichtung bzw. die Personenschleuse zuständige Person, die das bzw. die Wäschestücke aus dem Schleusenraum entfernt. Letztgenannte Person korrigiert daraufhin den Bestand an Wäschestücken in dem Wäschemagazin entsprechend.

Bei einer entsprechend schnell arbeitenden erfindungsgemäßen Kontrollvorrichtung ist es ebenfalls möglich, die zuvor genannten Sperrorgane gleichzeitig in einen Entsperrzustand zu steuern, woraufhin die zuvor genannte Erfassung der Wäschestückidentifikationsdaten während des Passierens des Schleusenraumes erfolgen kann, um die Zugangspassierberechtigung des Benutzers unter Mitführung von Wäschestücken ermitteln zu können. Entfällt diese während des Passierens des Schleusenraumes kann die jeweilige Zugangssperre vor Abschluss des Passierens des Zugangs gesperrt werden, um einen unzulässigen Transfer von Wäschestücken zu unterbinden.

Zur weiteren Vereinfachung insbesondere der Erfassung von Wäschestückidentifikationsdaten und Steigerung der Zuverlässigkeit ist in einer weiteren vorteilhaften Weiterbildung der Erfindung vorgesehen, dass die Mittel zur Erfassung von Benutzeridentifikationsdaten bzw. die ersten Erfassungsmittel zur Erfassung der Wäschestückidentifikationsdaten derart eingerichtet und ausgebildet sind, dass in Verwendungsposition der Zugangsvorrichtung eine Erfassung der Wäscheidentifikationsdaten von Wäschestücken bzw. Erfassung von Benutzeridentifikationsdaten für den Transfer aus bzw. in das Wäschemagazin zwischen dem dem Wäschemagazin zugewandten Sperrorgan und dem dem Wäschemagazin abgewandte Sperrorgan erfolgt.

Dadurch entsteht der Vorteil, dass der Bereich, in dem eine Erfassung von Wäschestückidentifikationsdaten bzw. Benutzeridentifikationsdaten erfolgt, eingrenzt ist, wodurch ein Erfassungsvorgang schneller und gleichfalls zuverlässig erfolgen kann. Demgemäß ist es erfindungsgemäß möglich, dass die Mittel zur Erfassung von Benutzeridentifikationsdaten auf gleiche Art und Weise wie die ersten bzw. zweiten Erfassungsmittel zur Erfassung von Wäschestückidentifikationsdaten eingerichtet und ausgebildet sind. Demzufolge kann eine entsprechende Erkennung beispielsweise und insbesondere mittels elektromagnetischer Strahlung vorzugsweise innerhalb des Schleusenraumes erfolgen, wodurch sich Einsparungen in den Kosten für Leseeinheiten ergeben, so dass es ebenfalls erfindungsgemäß möglich ist, eine gemeinsame Leseeinheit für die Erfassung von Wäschestückidentifikationsdaten wie auch für die Erfassung von Benutzeridentifikationsdaten zu verwenden.

Die Identifikationsmedien für eine Benutzererkennung können vor diesem Hintergrund auch an der Kleidung des entsprechenden Benutzers angeordnet sein.

Insbesondere zur Erhöhung der Präzision der Erfassung der Wäschestückidentifikationsdaten kann eine entsprechende Ausgestaltung der Wandungen des Schleusenraumes bzw. dessen Sperrorgane beitragen. Dabei können zunächst die Wandungen des Schleusenraumes auf unterschiedliche Art und Weise ausgeführt sein.

Dazu sieht eine vorteilhafte Weiterbildung der Erfindung vor, dass der Schleusenraum von seitlichen Wandungen begrenzt ist, wobei die seitlichen Wandungen des Schleusenraumes bzw. die Sperrorgane zur Konzentration der elektromagnetischen Strahlung für die Erfassung von Benutzeridentifikationsdaten bzw. die Erfassung von Wäschestückidentifikationsdaten innerhalb des Schleusenraumes eingerichtet und ausgebildet sind.

Beispielsweise und insbesondere sind diese wenigstens abschnittsweise durch Glas, wie es beispielsweise und insbesondere auch Sicherheitsglas bzw. Plexiglas sein kann, gebildet, um insbesondere einer übermäßig wahrgenommenen Raumenge von Personen mit klaustrophobischen Empfinden entgegenzuwirken.

Ferner kann dadurch der Schleusenraum von außen einsehbar ausgebildet sein, um insbesondere Notfallsituationen schnell durch außen stehende Personen erkennbar zu machen.

Dies unterstützend ist in einer weiteren vorteilhaften Weiterbildung der Erfindung vorgesehen, dass der Schleusenraum von seitlichen Wandungen begrenzt ist, wobei die seitlichen Wandungen des Schleusenraumes bzw. die Sperrorgane zur Konzentration der elektromagnetischen Strahlung für die Erfassung von Benutzeridentifikationsdaten bzw. die Erfassung von Wäschestückidentifikationsdaten innerhalb des Schleusenraumes eingerichtet und ausgebildet sind.

Erfindungsgemäß ist damit ebenfalls umfasst, dass ebenfalls Strahlungen von anderen Strahlungsquellen, die die Erfassung der Wäschestückidentifikationsdaten bzw. Benutzeridentifikationsdaten ungewünscht beeinflussen können, durch eine entsprechende Ausgestaltung der Wandungen bzw. Sperrorgane abgeschirmt werden können.

Dies kann beispielsweise durch das Material zur Bildung der Wandungen bzw. Sperrorgane bzw. deren Aufbau erfolgen. Ferner kann eine Beschichtung vorgesehen sein, mit der die Oberflächen der Wandungen bzw. Sperrorgane zur Konzentration von elektromagnetischer Strahlung in den Schleusenraum wenigstens abschnittsweise beschichtet sein können. Dazu existieren eine Vielzahl an Möglichkleiten, durch die Art des Aufbaus und die Verwendung von Werkstoffen für die Wandungen bzw. Sperrorgane (z.B. in einer Ausbildung als Tür) eine entsprechende Konzentration der elektromagnetischen Strahlung zu erreichen.

Eine erfindungsgemäße Kontrollvorrichtung kann für viele Arten von Wäschestücken eingesetzt werden und ist damit zur Verwendung in verschiedenen Anwendungsfeldern und Einrichtungen geeignet. Eine bevorzugte Verwendung der Erfindung liegt im Bereich der Berufsbekleidungslogistik, so dass erfindungsgemäß ein Verwendung einer erfindungsgemäßen Kontrollvorrichtung, wie sie zuvor beschrieben wurde, für ein Wäschemagazin zur Kontrolle des Transfers von Berufsbekleidungsstücken aus bzw. in das Wäschemagazin berücksichtigt ist.

Berufsbekleidungsstück können jegliche Art von Bekleidungsstücken bilden, wie es Kopfbedeckungen, Ober- und Unterbekleidung, Schuhe und dergleichen sein können.

Darüber hinaus bietet sich eine Verwendung der Erfindung in hygiene-sensiblen Bereichen an, in denen die Verfügbarkeit sauberer Wäsche- bzw. Berufsbekleidungsstücke für den Betrieb der Einrichtung bzw. Organisation entscheidend ist. Daher ist erfindungsgemäß eine Verwendung einer erfindungsgemäßen Kontrollvorrichtung für ein Wäschemagazin zur Kontrolle des Transfers von Berufsbekleidungsstücken aus bzw. in das Wäschemagazin einer Einrichtung des Gesundheitswesens vorgesehen.

Erfindungsgemäß ist eine Verwendung eine erfindungsgemäße Kontrollvorrichtung, wie diese u.a. zuvor beschrieben wurde, für eine Wäschemagazin eines Krankenhaus als prädestiniert vorgesehen. Die Wäschestücke sind dabei vorzugsweise Kleidungsstücke der Berufsbekleidung für Ärzte, Gesundheits- und Krankenpflegepersonal, sowie Fachpflegepersonal im Operationsdienst. Demgemäß werden durch ein Wäschemagazin entsprechend hygienesensible Wäschestücke bzw. Berufsbekleidungstücke bevorratet, wobei ein Transfer der hygiene-sensible Wäschestücke bzw. Berufsbekleidungstücke in bzw. aus dem Wäschemagazin mittels einer erfindungsgemäßen Kontrollvorrichtung erfolgt.

Darüber hinaus sind erfindungsgemäß für ein mögliches Verfahren für den Betrieb einer einer erfindungsgemäßen Kontrollvorrichtung Kontrolle des Transfers von Wäschestücken aus bzw. in ein Wäschemagazin beispielsweise und insbesondere folgende Schritte vorsehen:

Durch die Mitteln zur Erfassung von Benutzeridentifikationsdaten werden in einem ersten Schritt Benutzeridentifikationsdaten für eine konkret zugangsbegehrende Person erfasst. Daraufhin wird durch die Steuerungseinrichtung ermittelt, ob die Person grundsätzlich passierberechtigt ist.

In einem zweiten Schritt wird ermittelt, ob die Person zum Transfer von Wäschestücken aus dem bzw. in das Wäschemagazin berechtigt ist. Dazu wird zunächst durch erste Erfassungsmittel zur Erfassung von Wäschestückidentifikationsdaten die Art bzw. die Anzahl der Wäschestücke zum Transfer von der Person in oder aus dem Wäschemagazin geprüft. Dabei prüft das System durch Zugriff auf einen Datenspeicher, die zulässige Anzahl der von der Person transferierbaren Wäschestücke.

Daraufhin wird durch die Steuerungseinrichtung geprüft, ob die Anzahl der zum Transfer von dem Benutzer vorhanden Wäschestücke eine zulässige Anzahl übersteigt.

Dazu bildet die Steuerungseinrichtung eine Differenz zwischen einer im Datenspeicher festgelegten Obergrenze der durch den Benutzer transferierbaren Wäschestücken und der von dem Benutzer bereits transferierten Wäschestücken. Die Differenz bildet die zulässige Anzahl der transferierbaren Wäschestücke durch den Benutzer.

Sofern der Benutzer grundsätzlich passierberechtigt für den Zugang ist und die Anzahl der zum Transfer vorhandenen Wäschestücke kleiner oder gleich der Anzahl der für ein Passieren des Zugangs zugelassener Wäschestücke ist, werden das bzw. die Sperrorgane der Zugangsvorrichtung von der Steuerungseinrichtung zum Entsperren des Zugangs in einen Entsperrzustand gesteuert.

Andernfalls wird/werden der/die Sperrorgane der Zugangsvorrichtung von der Steuerungseinrichtung zum Sperren des Zugangs in einen Sperrzustand gesteuert. Dabei ist es alternativ ebenfalls im Sinne der Erfindung möglich, dass die überschüssigen Wäschestücke keinen Einfluss auf eine Passierberechtigung nehmen und daher für die Ermittlung einer Zugangspassierberechtigung unberücksichtigt bleiben. Die entsprechend erfassten Wäschestückidentifikationsdaten werden den Benutzeridentifikationsdaten in dem Datenspeicher zugeordnet, so dass z.B. der Benutzer auf die zuviel entnommen Wäschestücke hingewiesen wird und dieser damit entsprechend bezüglich der Mehrentnahme an Wäschestücken sensibilisiert wird.

In einem weiteren Schritt wird der Datenspeicher aktualisiert, um die Anzahl der beim Passieren des Zugangs transferierten Wäschestücke zu speichern.

Als ein weiterer Schritt ist erfindungsgemäß vorgesehen, dass die retournierten Wäschestücke durch die zweiten Erfassungsmittel insbesondere hinsichtlich Art und Anzahl der Wäschestücke erfasst werden, die von dem bzw. für den Benutzer insbesondere zum Zwecke der Reinigung, retourniert werden/wurden.

Daraufhin wird der der Datenspeicher durch die Steuerungseinrichtung bezüglich der von der Person transferierten Wäschestücke korrigiert.

Schließlich kann die betreffende Person wenigstens die Anzahl der Wäschestücke dem Wäschemagazin entnehmen, die zuvor retourniert wurden.

Das erfindungsgemäße Verfahren berücksichtigt dabei bevorrechtigte Personen, die beispielsweise den Bestand des Wäschemagazins pflegen bzw. das Wäschemagazin betreuen oder verwalten und dazu gereinigte bzw. neue Wäschestücke dem Wäschemagazin zuführen bzw. für den bestimmungsgemäßen Gebrauch ungeeignete Wäschestücke aus dem Wäschemagazin entfernen.

Dazu ist vorgesehen, dass diese bevorrechtigte Personen als Benutzer den Zugang der Zugangseinrichtung für ein Passieren von Wäschestücken freischalten, so dass eine erfindungsgemäße Kontrollvorrichtung den Zugang passierenden Wäschestücke durch die ersten Erfassungsmittel zur Erfassung von Wäschestückidentifikationsdaten der Steuerungseinrichtung erfasst und in einem Datenspeicher gespeichert bzw. verwaltet werden, um den dort gespeicherten Bestand an Wäschestücke zu aktualisieren. Dadurch wird es möglich, dass die Logistik bezüglich der im Bestand gehaltenen Wäschestücke vereinfacht ist.

Zudem ist es möglich, dass die Wäschestücke für eine schnelle Zufuhr bzw. Entnahme in Gebinden in bzw. aus dem Wäschemagazin transferiert werden, woraufhin das Gebinde entsprechend für die Erfassung durch die Mittel zur Erfassung von Wäschedaten gekennzeichnet werden kann und diese Kennzeichnung ggf. der Plausibilitätsprüfung dienen kann.

Darüber hinaus ist ein einer weiteren vorteilhaften Weiterbildung des erfindungsgemäßen Verfahrens vorgesehen, dass die Wäschestückidentifikationsdaten von einen am Wäschestück Informationsmedium mittels elektromagetischer Strahlung erfasst werden.

An die zuvor genannten Schritte können sich erfindungsgemäß weitere Schritte anfügen bzw. diese Schritte ergänzen. Desweiteren ist erfingungsgemäß die zuvor genannte Reihenfolge der Schritte veränder-, reduzier- und erweiterbar wie auch in ihrer Reihenfolge anpassbar.

Ergänzungen zu den vorstehenden Ausführungen ergeben sich im Weiteren anhand der Zeichnung und der dazu gehörigen Beschreibung. Daher wird die Erfindung anhand der beigefügten Zeichnung näher erläutert, in der Ausführungsbeispiele einer erfindungsgemäßen Kontrollvorrichtung bzw. dessen erfindungsgemäße Bestandteile dargestellt sind, die durch ein erfindungsgemäßes Verfahren betrieben werden können. Dabei bilden alle beanspruchten beschriebene und in der Zeichnung dargestellten Merkmale für sich genommen, sowie in beliebiger Kombination miteinander den Gegenstand der Erfindung, unabhängig von ihrer Zusammenfassung in den Ansprüchen und deren Rückbezügen sowie unabhängig von ihrer Beschreibung bzw. Darstellung in der Zeichnung.

Die Figuren der Zeichnung zeigen die zum Verständnis beitragenden Bestandteile der Ausführungsbeispiele einer erfindungsgemäßen Kontrollvorrichtung in jeweils einer dem besseren Verständnis angepassten bzw. vereinfachten Darstellung. Die Darstellungen sind daher stark schematisch und folglich weder maßstabs- noch detailgetreu, sondern sind zur besseren Übersicht auf die das Verständnis unterstützenden Elemente bzw. Bestandteile reduziert.

In den Figuren kennzeichnen gleiche Bezugszeichen, gleiche Bestandteile bzw. Elemente. Ferner sind aus Gründen einer besseren Übersicht nicht stets alle Bezugszeichen in den Figuren eingetragen, wobei die Ausführungen analog zu den entsprechend gleichen Bestandteilen gelten.

Es zeigt:
- Fig. 1: ein erstes Ausführungsbeispiel einer erfindungsgemäßen Kontrollvorrichtung, die in stark schematischer Darstellungsweise einen Grundaufbau verdeutlicht,
- Fig. 2a bis Fig. 2e: das Ausführungsbeispiel einer erfindungsgemäßen Kontrollvorrichtung aus Fig 1 in stark schematischer Darstellungsweise in verschiedenen exemplarischen Betriebszuständen in gleicher Darstellungsart wie in Fig. 1,
- Fig. 3: ein zweites Ausführungsbeispiel einer erfindungsgemäß Kontrollvorrichtung in gleicher Darstellungsweise wie sie in Fig. 1 gewählt ist.

Im Folgenden wird der Begriff Kontrollvorrichtung auch zur abkürzenden Schreibweise zur Bezeichnung eines Ausführungsbeispiel einer erfindungsgemäßen Kontrollvorrichtung verwendet.

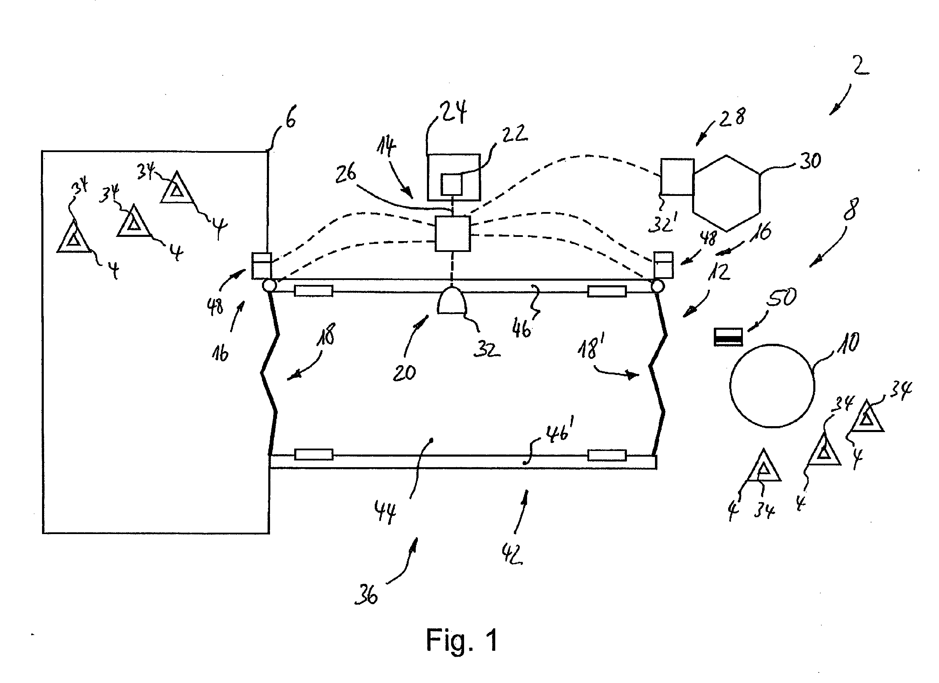

Fig. 1 zeigt ein erstes Ausführungsbeispiel einer erfindungsgemäßen Kontrollvorrichtung 2 zur Kontrolle des Transfers von Wäschestücken 4 (in den Figuren einheitlich mit dem Bezugszeichen 4 gekennzeichnet) aus bzw. in ein Wäschemagazin 6 in einer schematischen Draufsicht.

Das Ausführungsbeispiel einer erfindungsgemäßen Kontrollvorrichtung 2 (im folgenden verkürzt auch als Kontrollvorrichtung 2 bezeichnet) ist mit einer Zugangseinrichtung 8 ausgestattet, die in Verwendungsposition einen für einen Benutzer 10 bidirektional passierbaren Zugang 12 zum Wäschemagazin 8 aufweist.

Ferner sieht die Kontrollvorrichtung 2 eine Steuerungseinrichtung 14 vor, die wiederum Mittel 16 zur Erfassung von Benutzeridentifikationsdaten des Benutzers 10 aufweist, um mittels der erfassten Benutzeridentifikationsdaten eine Zugangspassierberechtigung des Benutzer 10 für ein Passieren des Zugangs 12 zu ermitteln.

Dabei ermöglicht es eine Steuerungseinrichtung 14, ein Sperrorgan 18, 18' der Zugangseinrichtung 8 in Abhängigkeit der Zugangspassierberechtigung des Benutzers 10 zwischen einem Sperrzustand, in dem der Zugang 12 durch das Sperrorgan 18,18' zum Passieren gesperrt ist, und einem Entsperrzustand, in dem der Zugang 12 durch das Sperrorgan 18,18' zum Passieren entsperrt ist, zu steuern, um die Kontrollvorrichtung 2 in verschiedenen Betriebsmodi betrieben werden können. Die Steuerungseinrichtung 14 ist mit dem betreffenden Ausstattungsbestandteil der Kontrollvorrichtung (u.a. 16, 18, 18', 12, 20, 22, 24, 48) entsprechend mit Daten- bzw. Signalleitung verbunden, um diese ansteuern bzw. mit ihr in Datenverbindung treten zu können. Die für den Datentransfer bzw. die Signalleitung benötigten Signal- bzw. Datenleitungen sind in den Figuren durch gestrichelte Linien dargestellt und zum Teil nicht näher gekennzeichnet bzw. erläutert, da sie lediglich die Zugehörigkeit zu der Steuerungseinrichtung 14 symbolisieren sollen.

Ferner weist die Steuerungseinrichtung 14 erste Erfassungsmittel 20 auf, die zur automatisierten und berührungslosen Erfassung von Wäschestückidentifikationsdaten einzelner Wäschestücke 4 aus einer Mehrzahl in wahlfreier Anordnung zueinander und wahlfreier Zusammenstellung den Zugang 12 passierender Wäschestücke 4 eingerichtet und ausgebildet sind.

Die Steuerungseinrichtung 14 erstellt eine Datenbeziehung zwischen den erfassten Daten, indem sie die erfassten Wäschestückidentifikationsdaten den erfassten Benutzeridentifikationsdaten zuordnet und diese Zuordnung in einem Datenspeicher 22 verwaltet.

Der Datenspeicher 22 ist in diesem Ausführungsbeispiel einer erfindungsgemäßen Kontrollvorrichtung 2 gebildet in einem Informationssystem bzw. Warenwirtschaftssystem 24 eines Krankenhauses, durch das zentral weitere Vorgänge unabhängig von einer Kontrollvorrichtung 2 verwaltet werden.

Für den Datentransfer zwischen Datenspeicher 22 und Steuerungseinrichtung 14 sieht die Kontrollvorrichtung 2 entsprechende Datenschnittstellen bzw. Datenanbindung 26 vor, um die von der Steuerungseinrichtung 14 erfassten Benutzeridentifikationsdaten bzw. Wäschestückidentifikationsdaten sowie daraus abgeleitete Daten bzw. die logistischen Vorgänge dokumentierende Daten in den Datenspeicher 22, insbesondere zur Verwaltung der Zuordnungen, schreiben bzw. verändern bzw. zur weiteren informationstechnischen Verarbeitung auslesen zu können.

Ferner weist die Steuerungseinrichtung 14 zweite Erfassungsmittel 28 auf, die für eine berührungslose und automatisierte Erfassung der Wäschestückidentifikationsdaten von über eine Rückgabeeinrichtung 30 retournierter Wäschestücke 4 eingerichtet und ausgebildet ist.

In diesem Ausführungsbeispiel einer erfindungsgemäßen Kontrollvorrichtung 2 ist die Rückgabeeinrichtung 30 nach Art eines Containers gebildet, der eine Einwurfklappe (nicht gezeigt) aufweist, über die die Wäschestücke 4 in den Container eingebracht werden können. Bei Betätigung der Einwurfklappe werden die zweiten Erfassungsmittel 28 mittels einer Lichtschranke (nicht gezeigt) aktiviert, woraufhin die Wäschestückidentifikationsdaten durch diese erfasst werden. Dabei erfolgt eine Erfassung aller im Container befindlichen Wäschestücke 4, so dass die neu zugeführten Wäschestücke 4 durch eine Differenzanalyse der erfassten Wäschestückidentifikationsdaten vor Einbringung der Wäschestücke 4 in den Container gegenüber der erfassten Wäschestückidentifikationsdaten nach den neu eingebrachten Wäschestücke 4 ermittelt werden kann.

Die so ermittelte Differenz wird dem entsprechenden Benutzer 10 zugeordnet, was durch die Zuordnung der Wäscheidentifikationsdaten zu den Benutzeridentifikationsdaten erfolgt. Dazu sind die Wäscheidentifikationsdaten mittels einer individuellen Identifikationsnummer eindeutig einem bestimmten Wäschestück 4 zugeordnet. Die weiteren Daten können somit dem Datenspeicher 22 entnommen werden. Durch Rückgabe der Wäschestücke 4 über die Rückgabeeinrichtung 30 verringert sich die entsprechende Anzahl der im Datenspeicher 22 gespeicherten bzw. verwalteten Wäschestücke 4, wie sie dem betreffenden Benutzer 10 zugeordnet sind.

Demgemäß ordnet die Steuerungseinrichtung 14 nach erfolgter Rückgabe der Wäschestücke 4 die Wäschestückidentifikationsdaten der retournierten Wäschestücke 4 den Benutzeridentifikationsdaten des Benutzers 10 zu und verwaltet diese Zuordnung (bzw. Beziehung) - wie bereits beschrieben - in dem Datenspeicher 22. Dabei ist entsprechend der vorstehenden Ausführungen vorgesehen, dass durch die Rückgabe von Wäschestücken 4 der betreffende Benutzer 10 eine entsprechende Anzahl von gereinigten bzw. neuen Wäschestücke 4 gleicher Art dem Wäschemagazin 6 entnehmen darf.

Die Rückgabeeinrichtung 30 ist in diesem Ausführungsbeispiel einer erfindungsgemäßen Kontrollvorrichtung 2 außerhalb des Wäschemagazins 6 wie auch außerhalb des Zugangs 12 zum Wäschemagazin 6, jedoch im Nahbereich zur Zugangseinrichtung 8 angeordnet, um eine Rückgabe/Retour von Wäschestücken 4 für den Benutzer 10 zur Benutzung einer erfindungsgemäßen Kontrollvorrichtung 2 zu vereinfachen.

Zudem weisen sowohl die ersten Erfassungsmittel 20 wie auch die zweiten Erfassungsmittel 28 jeweils eine Leseeinheit 32, 32' zur Erfassung von mittels elektromagnetischer Strahlung übertragener Wäschestückidentifikationsdaten auf, die auf einem an dem Wäschestück 4 angeordneten maschinenauslesbaren Informationsmedium 34 (einheitlich mit dem Bezugszeichen 34 gekennzeichnet) gespeicherten sind.

In diesem Ausführungsbeispiel einer erfindungsgemäßen Kontrollvorrichtung 2 ist das Informationsmedium 34 ein Ultrahochfrequenz-RFID-Transponder, der durch die entsprechende Leseeinheit 32, 32' zur Erfassung der auf dem Informationsmedium 34 gespeicherten Wäschestückidentifikationsdaten eingerichtet und ausgebildet ist. Die Leseeinheit 32, 32' ist damit als RFID-Leseeinheit bzw. RFID-Leser ausgebildet.

Darüber hinaus sieht die Kontrollvorrichtung 2 vor, dass die Zugangseinrichtung 8 als Vereinzelungseinrichtung 36 für einen personenseparierenden Zugang 12 zum Wäschemagazin 6 eingerichtet und ausgebildet ist, wobei zwei Sperrorgane 18, 18' für eine Separation der Benutzer 10 für ein Passieren des Zugangs 12 zeitgleich durch lediglich einen Benutzer 10 (sowie ggf. mitgeführter Wäschestücke 4) vorgesehen und entsprechend eingerichtet und ausgebildet sind.

Dazu ist die Vereinzelungseinrichtung 36 (u.a. in anderen Anwendungsbereichen auch bekannt als Vereinzelungsanlage) bei der in Fig. 1 dargestellten Kontrollvorrichtung 2 durch eine Personenschleuse 42 gebildet.

Die Personenschleuse 42 weist für den Zugang 12 zum Wäschemagazin 6 in Verwendungsposition der Zugangseinrichtung 8 bzw. der Personenschleuse 42 einen zwischen einem dem Wäschemagazin 6 zugewandten ersten Sperrorgan 18 und einen dem Wäschemagazin 6 abgewandten zweiten Sperrorgan 18' gebildeten Schleusenraum 44 auf, wobei die Sperrorgane 18,18' insbesondere für die Erfassung der Wäschestückidentifikationsdaten bzw. die Erfassung der Benutzeridentifikationsdaten durch die Steuerungseinrichtung 14 , insbesondere unabhängig voneinander, zwischen einem Sperrzustand und einem Entsperrzustand steuerbar sind.

Dadurch wird erreicht, dass ein Passieren des Zugangs 12 durch einen Benutzer 10, insbesondere unter Mitführung von Wäschestücken 4, auf unkontrollierte Art und Weise verhindert ist.

In diesem Ausführungsbeispiel einer erfindungsgemäßen Kontrollvorrichtung 2 ist vorgesehen, dass die Sperrorgane 18,18' unabhängig voneinander angesteuert werden können, um den Zugang 12 für ein Passieren des Benutzers 10 bzw. von Wäschestücken 4 durch die Sperrorgane 18,18' zu sperren bzw. zu entsperren.

Dabei ist die Leseeinheit 32 der ersten Erfassungsmittel 20 derart eingerichteten und ausgebildet, dass in Verwendungsposition der Zugangsvorrichtung 8 bzw. der Personenschleuse 42 eine Erfassung der Wäscheidentifikationsdaten von Wäschestücken 4 für den Transfer aus bzw. in das Wäschemagazin 6 zwischen dem Wäschemagazin 6 zugewandten Sperrorgan 18 und dem Wäschemagazin 6 abgewandte Sperrorgan 18' erfolgt.

Zur Unterstützung der Erfassung von Wäschestückidentifikationsdaten durch die Steuerungseinrichtung 14 ist erfindungsgemäß vorgesehen, dass der Schleusenraum 44 von seitlichen Wandungen 46,46' begrenzt ist, wobei die seitlichen Wandungen 46,46' des Schleusenraumes 44 wie auch die Sperrorgane 18,18' zur Konzentration der elektromagnetischen Strahlung für die Erfassung von Benutzeridentifikationsdaten bzw. die Erfassung von Wäschestückidentifikationsdaten innerhalb des Schleusenraumes 44 eingerichtet und ausgebildet sind. Dazu sind die dem Schleusenraum 44 zugewandten Oberflächen der Wandungen bzw. Sperrorgane zur wenigstens abschnittsweisen Ausbildung einer Metallschicht auf diesen Oberflächen entsprechend bedampft.

Dadurch wird erfindungsgemäß erreicht, dass die elektromagnetische Strahlung im Wesentlichen innerhalb des Schleusenraumes 44 gehalten wird, so dass eine Streuung der elektromagnetischen Strahlen nach außerhalb des Schleusenraumes 44 minimiert ist. Darüber hinaus ist dadurch erreicht, dass Störfelder die Erfassung durch die Steuerungseinrichtung 14 der erfindungsgemäßen Kontrollvorrichtung 2 nicht bzw. lediglich vernachlässigbar gering eine Erfassung der genannten Daten stören.

Darüber hinaus sieht die Kontrollvorrichtung 2 Mittel 16 zur Erfassung von Benutzeridentifikationsdaten vor, die wenigstens eine (in diesem Ausführungsbeispiel einer erfindungsgemäßen Kontrollvorrichtung 2 zwei) Datenerfassungseinheit 48 (einheitlich mit dem Bezugszeichen 48 gekennzeichnet) für das Erfassen eines maschinenauslesbaren Identifikationsmediums 50 aufweisen, auf dem die Benutzeridentifikationsdaten gespeichert sind, um auf diese Art und Weise den Benutzer 10 für die Ermittlung der Zugangspassierberechtigung identifizieren zu können.

Im Rahmen der Benutzererkennung ist es erfindungsgemäß vorgesehen, dass ein Grad der Identifizierung eines Benutzers 10 ausreichend ist, der eine Ermittlung einer Zugangspassierberechtigung für den Benutzer 10 erlaubt.

Die in Fig. 1 dargestellt Kontrollvorrichtung 2 ist zur Verwendung in einem Krankenhaus eingerichteten und ausgebildet, so dass als Wäschestücke 4 Berufsbekleidungsstücke , insbesondere von Pflegepersonal und Ärzten, innerhalb eines Wäschemagazins 6 durch die Kontrollvorrichtung 2 kontrolliert werden können.

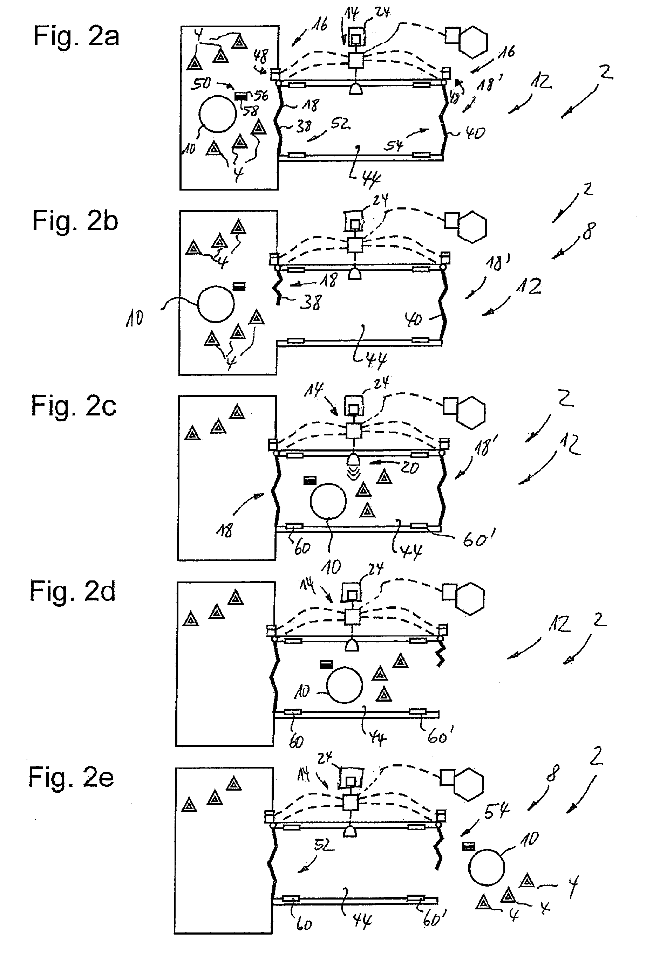

Des weiteren ist die in Fig.1 gezeigten Kontrollvorrichtung 2 mit weiteren Bestandteilen ausgestattet, die im Folgenden anhand der Fig. 2 exemplarisch gezeigten Betriebszustände der Kontrollvorrichtung 2 am Beispiel des Passierens des Zugangs 6 in das Wäschemagazin 8 hinein verkürzt und stellvertretend für weitere mögliche Betriebsmodi einer erfindungsgemäßen Kontrollvorrichtung 2 erläutert werden sollen.

Die Darstellung in Fig.2 ist wiederum schematisch gewählt und auf die das Verständnis unterstützenden Elemente bzw. Bestandteile zum Betrieb einer erfindungsgemäßen Kontrollvorrichtung 2 reduziert.

In Fig. 2 a ist zunächst ein Sperrzustand der Sperrorgane 18, 18' der Kontrolleinrichtung 2 gezeigt.

Die Sperrorgane 18,18' sind jeweils durch ein Tür 38,40 die in diesem Ausführungsbeispiel eine Falttür ist, realisiert, die voneinander unabhängig zum Schließen und Öffnen einer jeweiligen Zugangsöffnung 52,54 des Zugangs 12 durch die Steuerungseinrichtung 14 steuerbar sind, damit ein Benutzer 10 bzw. Wäschestücke 4 in den bzw. aus dem Schleusenraum 44 gelangen können.

Um in den Schleusenraum 44 gelangen zu können, weisen die Mittel 16 zur Erfassung von Benutzerdaten jeweils eine Datenerfassungseinheit 48 (einheitlich mit dem Bezugszeichen 48 gekennzeichnet) angeordnet an den Sperrorganen 18,18' auf, die außerhalb des Schleusenraumes 44 angeordnet sind.

Durch die Datenerfassungseinheiten 48 wird ein Auslesen eines maschinenauslesbaren Identifikationsmediums 50 realisiert, dass in diesem Ausführungsbeispiel einer erfindungsgemäßen Kontrollvorrichtung 2 als eine Chipkarte 56 mit einem Magnetstreifen 58 ausgebildet ist, auf dem die Benutzeridentifikationsdaten gespeichert sind.

Das Identifikationsmedium 50 wird durch eine Datenerfassungseinheit 48 mittels ihres Magnetstreifen 58 ausgelesen. Die so ermittelten Benutzeridentifikationsdaten werden durch die Steuerungseinrichtung 14 verarbeitet, um eine grundsätzliche Zugangspassierberechtigung für den Benutzer 10 zu ermitteln.

Daraufhin wird zunächst das Sperrorgane 18 mittels der Steuerungseinrichtung 14 durch automatisches Öffnen der sie bildenden Tür 38 zum Wechsel in einen Entsperrzustand gesteuert, wobei die Tür 38 benachbart ist zu der Datenerfassungseinheit 48, über die die Benutzeridentifikation erfolgt ist.

Daraufhin gelangt der Benutzer 10 mit den Wäschestücken 4 in den Schleusenraum 44, wobei das verbleibende Sperrorgan 18' gesperrt bleibt, wodurch ein Passieren des Zugangs 12 durch den Benutzer 10 nicht möglich ist und der Zugang 12 der Zugangseinrichtung 8 weiterhin nicht passierbar ist. Dadurch ist durch Entsperren eines der Sperrorgane 38,40 zunächst lediglich eine Teilentsperrung des Zugangs 12 erfolgt, woraufhin der Schleusenraum 44 für den Benutzer 10 zugänglich ist. Dies ist in Fig. 2b gezeigt.

Nach Eintritt der Person 10 in den Schleusenraum 44 wird das Sperrorgan 18', über die der Benutzer 10 in den Zugang 12 gelangt ist, durch die Steuerungseinrichtung in einen Sperrzustand 14 gesteuert, so dass die Erfassung der Wäschestückidentifikationsdaten für die Erkennung der Wäschestücke 4 erfolgen kann. Dies ist in Fig. 2c veranschaulicht. Ein Einbringen von Wäschestücke 4 oder ein Eintreten eines Benutzers 10 in den Schleusenraum 44 erkennt zunächst eine im Nahbereich des jeweiligen Sperrorgans 18,18' angeordnete Lichtschranke 60, 60', durch die Wäschestücke 4 bzw. die Person 10 in den Schleusenraum 44 gelangt sind.

Daraufhin werden die ersten Erfassungsmittel 20 zur Erfassung der Wäschestückidentifikationsdaten durch die Steuerungseinrichtung 14 zur Erfassung der entsprechenden Daten aktiviert. Danach erfolgt die Erfassung der Wäschestückidentifikationsdaten durch die ersten Erfassungsmittel 20.

Nachdem die Erfassung der Wäschestückidentifikationsdaten abgeschlossen ist, wird durch die Steuerungseinrichtung 14 eine Zugangspassierberechtigung auf der Grundlage der erfassten Benutzeridentifikationsdaten und der erfassten Wäschestückidentifikationsdaten ermittelt, so dass bei Unterschreiten einer zulässigen Anzahl von Wäschestücken 4 der Zugang 12 für ein Passieren durch den Benutzer 10 ermöglicht wird, woraufhin die das Sperrorgan 18' durch die Steuerungseinrichtung 14 gesteuert in einen Entsperrzustand wechselt. Dies ist in Fig. 2d gezeigt.

Danach registriert eine Lichtschranke 60' an dem zuletzt entsperrten Sperrorgan 18', dass der Zugang 12 bzw. der Schleusenraum 44 durch den Benutzer 10 bzw. das Wäschestück 4 / die Wäschestücke 4 verlassen wurde. Dies in Fig. 2e gezeigt. Sodann schließt durch die Steuerungseinrichtung 14 gesteuert die geöffnete Tür 38, wobei die Sperrorgane 18,18' die jeweilige Zugangsöffnung 52,54 für einen Zutritt zum Schleusenraum 44 sperren und die Zugangseinrichtung 8 durch die Steuerungseinrichtung 14 gesteuert in einen Sperrzustand wechselt.

Anschließend bzw. zeitgleich steuert die Steuerungseinrichtung 14 die ersten Erfassungsmittel 20 zur Erfassung der Wäschestückidentifikationsdaten derart, dass diese in einen deaktiven Modus wechseln, in dem eine Erfassung von Wäschestückidentifikationsdaten innerhalb des Schleusenraumes 44 nicht erfolgt.

Auf diese Weise ist erreicht, dass insbesondere der Energiebedarf einer Kontrollvorrichtung 2 reduziert werden kann. Darüber hinaus ist erreicht, dass die elektromagnetischen Strahlung reduziert ist.

Anschließend steht die Kontrollvorrichtung 2 für ein weiteres Passieren bzw. Transferieren zur Verfügung, beispielsweise für ein Passieren in das Wäschemagazin 6 hinein, was analog der vorstehenden Beschreibung erfolgen kann.

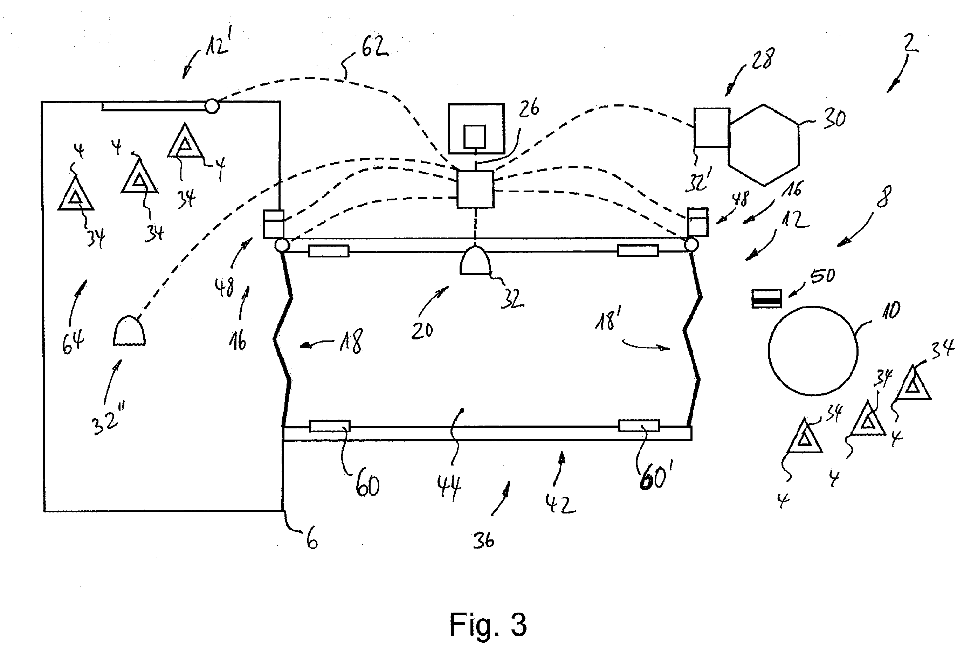

Fig. 3 zeigt ein zweites Ausführungsbeispiel einer erfindungsgemäßen Kontrollvorrichtung 2 (nachfolgend verkürzt auch als Kontrollvorrichtung 2 bezeichnet), wobei die nachfolgenden Ausführungen zur besseren Übersicht auf die Unterschiede zum ersten Ausführungsbeispiel konzentriert sind.

Die Kontrollvorrichtung 2 ist in diesem Ausführungsbeispiel mittels einer Daten- und Steuerungsverbindung 62 mit einer weiteren Zugangseinrichtung 12' verbunden, die lediglich zur Ermittlung und Prüfung einer grundlegende Zugangspassierberechtigung eines Benutzers 10 eingerichtet und ausgebildet ist, wobei eine Erfassung von Wäschestückidentifikationsdaten bei der weiteren Zugangseinrichtung 12' nicht erfolgt.

Innerhalb des Wäschemagazins 6 sieht die erfindungsgemäße Kontrollvorrichtung 2 eine weitere Leseeinheit 32" vor, um eine erweiterte Kontrolle des in dem Wäschemagazin 6 befindlichen Wäschevorrates 64 vornehmen zu können. Dadurch ist beispielsweise eine Plausiblitätsprüfung der an der Personenschleuse 42 ermittelten Daten bezüglich der erfassten Wäschestücke 4 möglich.

Die Erfindung ist in viele Richtungen und weiten Grenzen ausgestaltbar. Beispielsweise kann die Erfassung von Benutzeridentifikationsdaten auf gleiche Art und Weise erfolgen, wie bei den Wäschestücken 4, beispielsweise und insbesondere mittels RFID-Technologien. Dazu sind die entsprechenden Mittel 16 zur Erfassung von Benutzeridentifikationsdaten entsprechend auszubilden und einzurichten, wobei diese mit den ersten bzw. zweiten Erfassungsmitteln 20,28 von Wäschestückidentifikationsdaten auch identisch sein können.

Zudem ist es möglich, eine erfindungsgemäße Kontrollvorrichtung 2 in verschiedenen Modi zu betreiben, um beispielsweise die Zufuhr gereinigter Wäschestücke 4 in das Wäschemagazin 6 zu erleichtern, indem die Kontrollvorrichtung 2 die dem Wäschemagazin 6 über den Zugang 12 zugeführten Wäschestücke 4 erfasst und dadurch den in einem Datenspeicher 22 gespeicherten Bestand an Wäschestücken 4 aktualisiert.

## Patentansprüche

1. Kontrollvorrichtung zur Kontrolle des Transfers von Wäschestücken aus bzw. in ein Wäschemagazin,
mit einer Zugangseinrichtung (4), die in Verwendungsposition einen für einen Benutzer bidirektional passierbaren Zugang (6) zum Wäschemagazin (8) aufweist, wobei eine Steuerungseinrichtung Mittel zur Erfassung von Benutzeridentifikationsdaten des Benutzers aufweist, um mittels der erfassten Benutzeridentifikationsdaten eine Zugangspassierberechtigung des Benutzer für ein Passieren des Zugangs zu ermitteln,
so dass die Steuerungseinrichtung ein Sperrorgan der Zugangseinrichtung in Abhängigkeit der Zugangspassierberechtigung des Benutzers zwischen einem Sperrzustand, in dem der Zugang durch das Sperrorgan zum Passieren gesperrt ist, und einem Entsperrzustand, in dem der Zugang durch das Sperrorgan zum Passieren entsperrt ist, steuert,
**dadurch gekennzeichnet,**
**dass** die Steuerungseinrichtung (14) erste Erfassungsmittel (14) aufweist, die zur berührungslosen Erfassung von Wäschestückidentifikationsdaten einzelner Wäschestücke (4) aus einer Mehrzahl in wahlfreier Anordnung zueinander und wahlfreier Zusammenstellung den Zugang passierender Wäschestücke (4) eingerichtet und ausgebildet sind,
wobei die Steuerungseinrichtung die erfassten Wäschestückidentifikationsdaten den erfassten Benutzeridentifikationsdaten zuordnet und diese Zuordnung in einem Datenspeicher (22) verwaltet.

2. Kontrollvorrichtung nach Anspruch 1, **dadurch gekennzeichnet, dass** die Steuerungseinrichtung (14) zweite Erfassungsmittel (28) aufweist,
die für eine berührungslose Erfassung der Wäschestückidentifikationsdaten von über eine Rückgabeeinrichtung (30) retournierter Wäschestücke (4) eingerichtet und ausgebildet ist,
wobei die Steuerungseinrichtung (14) die Wäschestückidentifikationsdaten der retournierten Wäschestücke (4) den Benutzeridentifikationsdaten des Benutzers (10) zuordnet und diese Zuordnung in dem Datenspeicher (22) verwaltet.

3. Kontrollvorrichtung nach Anspruch 1 oder 2, **dadurch gekennzeichnet, dass** die ersten Erfassungsmittel (20) bzw. die zweiten Erfassungsmittel (28) jeweils wenigstens eine Leseeinheit (32,32',32") zur Erfassung von mittels elektromagnetischer Strahlung übertragener Wäschestückidentifikationsdaten aufweisen, die auf einem an dem Wäschestück (4) angeordneten maschinenauslesbaren Informationsmedium (40) gespeicherten sind, wobei das Informationsmedium insbesondere ein RFID-Transponder, vorzugsweise ein Ultrahochfrequenz-RFID-Transponder, ist und die Leseeinheit (32,32',32") insbesondere zur Erfassung der auf dem RFID-Transponder gespeicherten Wäschestückidentifikationsdaten eingerichtet und ausgebildet ist.

4. Kontrollvorrichtung nach einem der vorhergehenden Ansprüche , **dadurch gekennzeichnet, dass** die Zugangseinrichtung (8) zur wenigstens abschnittsweisen Bildung des Zugangs (12, 12') wenigstens eine Vereinzelungsvorrichtung (36) aufweist, bei der insbesondere wenigstens ein Sperrorgan (18, 18') für eine Separation der Benutzer (10) für ein Passieren des Zugangs (12, 12') eingerichtet und ausgebildet ist.

5. Kontrollvorrichtung nach einem der vorhergehenden Ansprüche, **dadurch gekennzeichnet, dass** die Vereinzelungseinrichtung (36) nach Art einer Personenschleuse (42) eingerichtet und ausgebildet ist, die für den Zugang (12,12') zum Wäschemagazin (6) in Verwendungsposition der Personenschleuse (42) einen zwischen einem dem Wäschemagazin (6) zugewandten ersten Sperrorgan (18) und einen dem Wäschemagazin (6) abgewandten zweiten Sperrorgan (18') gebildeten Schleusenraum (32) aufweist, wobei die Sperrorgane (18,18') insbesondere für die Erfassung der Wäschestückidentifikationsdaten durch die ersten Erfassungsmittel (20) bzw. die Erfassung der Benutzeridentifikationsdaten durch die Mittel (16) zur Erfassung von Benutzeridentifikationsdaten, insbesondere unabhängig voneinander, zwischen einem Sperrzustand und einem Entsperrzustand steuerbar sind.

6. Kontrollvorrichtung nach einem der vorhergehenden Ansprüche, **dadurch gekennzeichnet, dass** die Mittel (16) zur Erfassung von Benutzeridentifikationsdaten bzw. die ersten Erfassungsmittel (2) zur Erfassung der Wäschestückidentifikationsdaten derart eingerichtet und ausgebildet sind, dass in Verwendungsposition der Zugangseinrichtung (8) eine Erfassung der Wäscheidentifikationsdaten von Wäschestücken (4) für den Transfer aus bzw. in das Wäschemagazin (8) bzw. eine Erfassung der Benutzeridentifikationsdaten des Benutzers (10) für ein Passieren des Zugangs (12) zwischen dem dem Wäschemagazin (6) zugewandten ersten Sperrorgan (18) und dem dem Wäschemagazin (6) abgewandte zweiten Sperrorgan (18') erfolgen.

7. Kontrollvorrichtung nach Anspruch 3 und einem der Ansprüche 5 bis 6, **dadurch gekennzeichnet**, der Schleusenraum (44) von seitlichen Wandungen (46,46') begrenzt ist, wobei die seitlichen Wandungen (46,46') des Schleusenraumes (44) bzw. die Sperrorgane (18,18') zur Konzentration der elektromagnetischen Strahlung für die Erfassung von Benutzeridentifikationsdaten bzw. die Erfassung von Wäschestückidentifikationsdaten innerhalb des Schleusenraumes eingerichtet und ausgebildet sind.

8. Kontrollvorrichtung nach einem der vorhergehenden Ansprüche, **dadurch gekennzeichnet, dass** die Mittel (16) zur Erfassung von Benutzeridentifikationsdaten wenigstens eine Datenerfassungseinheit (48) für das Erfassen eines maschinenauslesbaren Identifikationsmediums (50) aufweisen, auf dem die Benutzeridentifikationsdaten gespeichert sind.

9. Verwendung einer Kontrollvorrichtung (2) nach einem der vorhergehenden Ansprüche für ein Wäschemagazin (6) zur Kontrolle des Transfers von Berufsbekleidungsstücken (4) aus bzw. in das Wäschemagazin (6).

10. Verwendung einer Kontrollvorrichtung (2) nach einem der Ansprüche 1 bis 10 für ein Wäschemagazin (6) zur Kontrolle des Transfers von Berufsbekleidungsstücken (4) aus bzw. in das Wäschemagazin (4) einer Einrichtung des Gesundheitswesens.
